# EUROPEAN PATENT SPECIFICATION

(11) **EP 1 586 598 B1**
(45) Date of publication and mention of the grant of the patent: **09.09.2009**
(21) Application number: 05076346.5
(22) Date of filing: 18.11.2002
(51) Int. Cl.: C08G 65/48, C08G 63/692, B01J 31/18, B01J 31/16

(54) **Process for preparation of polymeric, phosphorus-containing compositions**
Verfahren zur Herstellung von polymeren phosphorhaltigen Zusammensetzungen
Procédé de préparation des compositions polymériques qui contiennent du phosphore

(30) Priority: 26.11.2001 US 994097
(43) Date of publication of application: 19.10.2005
(62) Divisional of application: 02789715.6
(73) Proprietor: INVISTA Technologies S.à.r.l., 8001 Zürich (CH)
(72) Inventor: Radu, Nora S., Landenberg, PA 19350 (US); Tam, Wilson, Boothwyn, PA 19061 (US)
(74) Representative: Cockerton, Bruce Roger

(56) References cited:
- WO-A-01/21308
- WO-A-01/21684
- US-A- 6 121 184

## Description

### BACKGROUND OF THE INVENTION

### FIELD OF THE INVENTION

The present invention relates to the preparation of polymeric, phosphorus-containing ligand compositions. The present invention also relates to catalyst compositions involving a Group VIII metal in the presence of the polymeric ligands and use of such catalysts in hydrocyanation, isomerization, and hydroformylation reactions.

### DESCRIPTION OF THE RELATED ART

Phosphorus-based ligands are generally known in catalysis, finding use for a number of commercially important chemical transformations. Phosphorus-based ligands commonly encountered in catalysis include phosphines, phosphinites, phosphonites and phosphites. Monodentate phosphorous ligands, e.g. monophosphine and monophosphite ligands, are compounds that usually contain a single phosphorus atom that serves as an electron donor to a transition metal. Bidentate phosphorous ligands, e.g. bisphosphine, bisphosphinite, bisphosphonite, bisphosphite, and bis(phosphorus) ligands, in general, contain two phosphorus electron donor atoms and typically form cyclic chelate structures with transition metals.

Two catalytic reactions of particular industrial importance using phosphorous ligands are olefin hydrocyanation and isomerization of branched nitriles to linear nitriles. Phosphite and phosphinite ligands are particularly good ligands for both reactions. The hydrocyanation of ethylenically unsaturated compounds (olefins) using transition metal complexes with monodentate phosphite ligands is well documented in the prior art. See for example U. S. Pat. Nos. 3,496,215; 3,631,191; 3,655,723; 3,766,237; and 5,543,536. Bidentate phosphite ligands have also been shown to be particularly useful ligands in the hydrocyanation of activated ethylenically unsaturated compounds. See for example, J. Chem. Soc., Chem. Commun., 1991, 1292; J.Chem. Soc., Chem. Commun., 1991, 803; PCT Pat. App. WO 9303839; and U. S. Pat. Nos. 5,512,696; 5,723,641; and 5,688,986. Bidentate phosphinite and phosphonite ligands are described in U.S. Pat. Nos. 5,817,850; 5,523,453; and 5,693,843, and PCT Pat. Apps. WO 9964155, WO 9913983, WO 9946044, and WO 9843935.

Hydroformylation is another industrially useful process that utilizes catalysts made from phosphorus-containing ligands. The use of phosphine ligands, including diphosphines, is known for this purpose. The use of catalysts made from phosphite ligands is also known. Such catalysts usually contain a Group VIII metal. See for example, U. S. Pat. No. 5,235,113.

Recovery of the ligand and catalyst is important for a successful commercial process. Typical separation procedures to remove the product(s) from the catalyst and ligand involve extraction with an immiscible solvent or distillation. It is usually difficult to recover the catalyst and ligand quantitatively. For instance, distillation of a volatile product from a non-volatile catalyst results in thermal degradation of the catalyst. Similarly, extraction results in some loss of catalyst into the product phase. For extraction, one would like to be able to select and/or control the solubility of the ligand and catalyst to disfavor solubility in the product phase. These ligands and metals are often very expensive and thus it is important to keep such losses to a minimum for a commercially viable process.

One method to solve the problem of catalyst and product separation is to attach the catalyst to an insoluble support. Examples of this approach have been previously described, and general references on this subject can be found in "Supported Metal Complexes", D. Reidel Publishing, 1985; Acta Polymer., 1996, 47, 1; "Comprehensive Organometallic Chemistry", Pergamon Press, 1982, Chapter 55; J. Mol. Catal. A, 1995, 104, 17 and Macromol. Symp., 1994, 80, 241. Specifically, monophosphine and monophosphite ligands attached to solid supports are described in these references. Bisphosphine ligands have also been attached to solid supports and used for catalysis, as described in, for example, U. S. Pat. Nos. 5,432,289 and 5,990,318; J. Mol. Catal. A, 1996, 112,217; J.Chem. Soc., Chem. Commun., 1996, 653; J. Org. Chem., 1998, 63, 3137; Spec. Chem., 1998, 18, 224 and PCT Pat. App. WO 9812202. PCT Pat. Apps. WO 9906146 and WO 9962855 show the use of supported phosphorus ligands in hydrocyanation and hydroformylation reactions, respectively. Bisphosphite ligands have also been grafted to solid supports such as those described in U. S. Pat. No. 6,121,184. The solid support in these prior art examples can be organic, e.g., a polymer resin, or inorganic in nature.

Polymer-supported multidentate phosphorous ligands may be prepared by a variety of methods known in the art, as described in U. S. Pat. Nos. 4,769,498 and 4,668,651, PCT Pat. Apps. WO 9303839 and WO 9906146, and European Pat.Apps. EP 0864577 A2 and EP 0877029 A2. The prior art discloses side-chain polymers containing multidentate phosphorous ligands as pendant groups.

Another method to solve the problem of separating the catalyst from the reaction product is to copolymerize phosphorus-containing ligands with other non-ligand monomers to produce insoluble phosphorus-containing ligands. Examples of such polymer-immobilized phosphine ligands have been reported in J. Am. Chem. Soc., 2000, 122, 6217 and J. Org. Chem., 1986, 51, 4189. In addition, polymer-immobilized phosphine-phosphite ligands and their use in hydroformylation catalysis recently have been described in Bull. Chem. Soc. Jpn., 1999, 72, 1911; J. Am. Chem. Soc., 1998, 120, 4051; and European Pat. App. EP 0864577.

WO-A-01/21684 discloses a polymeric composition, a process for producing the composition, and a process for using the composition in, for example, hydrocyanation or isomerization. The composition comprises repeat units derived from (1) a carbonyl compound, a monomer, and phosphorochloridite; (2) phosphorus trichloride, a polyhydric alcohol, and an aromatic diol; or (3) combinations of (1) and (2) in which the monomer can be a polyhydric alcohol, an amine, combinations thereof. The composition can be produced by (1) contacting a carbonyl compound with the monomer to produce an intermediate and contacting the intermediate with phosphorochloridite; (2) contacting phosphorus trichloride with a second polyhydric alcohol under a condition sufficient to produce a phosphorus-containing polymer and contacting the phosphorus-containing polymer with an aromatic diol; or (3) contacting an N,N-dialkyl dichlorophosphoramidite with a second polyhydric alcohol to produce a polymer phosphoramidite, contacting the polymer phosphoramidite with an acid such as HCl to produce the phosphorus-containing polymer, which is then contacted with an aromatic diol.

WO-A-01/21308 discloses a composition and a process for using the composition in hydroformylation of unsaturated organic compounds. The composition comprises a Group VIII metal and a phosphite-containing polymer having repeat units derived from (1) a carbonyl compound, a monomer, and phosphochloridite; (2) phosphorus trichloride, a polyhydric alcohol, and an aromatic diol; or (3) combinations of (1) and (2). The process comprises contacting an unsaturated compound, in the presence of the composition disclosed herein, with a fluid comprising hydrogen.

U.S. Patent No. 6,121,184 discloses supported bis(phosphorus) ligands for use in a variety of catalytic processes, including the hydrocyanation of unsaturated organic compounds. Catalysts are formed when the ligands are combined with a catalytically active metal.

### BRIEF SUMMARY OF THE INVENTION

The first aspect of the present invention provides a method to produce a polymeric, phosphorus-containing composition by:
1. preparing a polymeric precursor by polymerization of a composition comprising at least one compound of Formula I and/or at least one compound of Formula II, 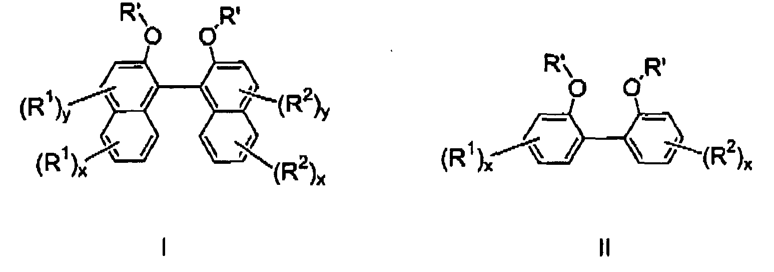 wherein:
   x = 0 to 4;
      y = 0 to 2;
      each R' is individually hydrogen or a hydroxyl protective group selected from alkyl (i.e., CH₃-), alkoxyalkyl (i.e., CH₃OCH₂-), carbonylalkyl (i.e., CH₃-C(O)-), and a crown ether formed by taking both R' groups together.
      Each R¹ and each R² are individually hydrogen, linear or branched alkyl, cycloalkyl, acetal, ketal, aryl, alkoxy, cycloalkoxy, aryloxy, ester, nitrile, fluorine, chlorine, bromine, perhaloalkyl, hydrocarbylsulfinyl, hydrocarbylsulfonyl, formyl, hydrocarbylcarbonyl or cyclic ether;
      provided at least two R¹ or at least two R² or at least one R¹ and at least one R² are capable of reacting with one another to cause aryl to aryl coupling of at least one compound of Formula I and/or at least one compound of Formula II; and
      wherein said aryl to aryl coupling is achieved by:
      (i) polymerization of a composition comprising at least one compound of Formula I and/or at least one compound of Formula II to form a carbon to carbon bond linking at least one compound of Formula I and/or at least one compound of Formula II; or
      (ii) copolymerization of a composition comprising at least one compound of Formula I and/or at least one compound of Formula II in the presence of an aryl comonomer having at least two R¹ groups or at least two R² groups or at least one R¹ group and at least one R² group capable of reacting with at least one compound of Formula I and/or at least one compound of Formula II; or
      (iii) copolymerization of a composition comprising at least one compound of Formula I and/or at least one compound of Formula II by a Friedel-Crafts reaction of at least one compound of Formula I and/or at least one compound of Formula II in the presence of at least one dialkylating or dibenzylating or diacylating comonomer; or
      (iv) copolymerization of at least one compound of Formula II with an aldehyde, and
   2. if R' is a hydroxyl protective group, converting R' to H or alkali metal or alkaline earth metal, and
   3. phosphonylating the product of step (1) if R' is other than a hydroxyl protective group, or phosphonylating the product of steps (1) and (2) if R' is a hydroxyl protective group, with at least one diaryloxyphosphite [-P(-O-Ar)₂] diarylphosphine [-P(Ar)₂], and/or aryl,aryloxyphosphinite [-P(Ar)(-O-Ar)], wherein each Ar is individually phenyl, substituted phenyl, naphthyl, or substituted naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide.

The second aspect of the present invention provides a polymeric, phosphorus-containing composition made as described above in aspect one.

The third aspect of the present invention provides a method to produce a polymeric, phosphorus-containing composition by polymerization of a composition comprising at least one substituted phosphonylated 2,2'-dihydroxyl-1,1'-binaphthalene as shown in Formula III and/or at least one substituted phosphonylated 2,2'-dihydroxyl-1,1'-biphenylene as shown in Formula IV: 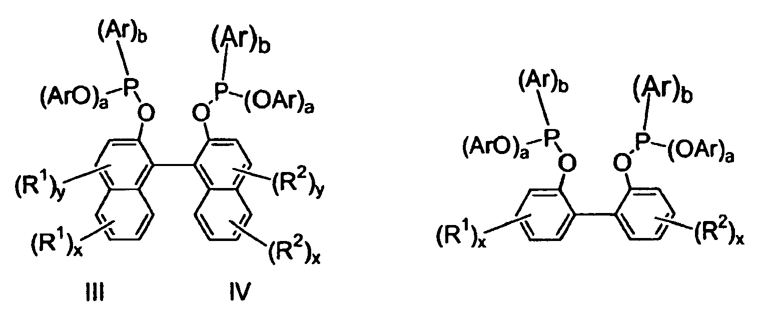 wherein:
x = 0 to 4;
y = 0 to 2;
a and b are individually either 0, 1, or 2, provided a+b = 2;
each Ar is individually phenyl or naphthyl, provided that the two Ar groups that are directly or indirectly bonded to the same phosphorus atom may be linked to each other by a linking unit selected from the group consisting of direct bond, alkylidene, secondary or tertiary amine, oxygen, sulfide, sulfone, or sulfoxide;
each Ar can be further substituted with C₁ to C₂₀ branched or straight chain alkyl, C₁ to C₂₀ cycloalkyl, C₆ to C₂₀ aryl, acetal, ketal, alkoxy, cycloalkoxy, aryloxy, formyl, ester, fluorine, chlorine, bromine, perhaloalkyl, hydrocarbylsulfinyl, hydrocarbylsulfonyl, hydrocarbylcarbonyl or cyclic ether;
each R¹ and each R² are individually hydrogen, linear or branched alkyl, cycloalkyl, acetal, ketal, aryl, alkoxy, cycloalkoxy, aryloxy, ester, amine, boronic acid, boronic ester, nitrile, fluorine, chlorine, bromine, perhaloalkyl, hydrocarbylsulfinyl, hydrocarbylsulfonyl, formyl, hydrocarbylcarbonyl or cyclic ether;
provided at least two R¹ groups or at least two R² groups or at least one R¹ group and at least one R² group are capable of reacting with one another to cause aryl to aryl coupling of at least one compound of Formula III and/or at least one compound of Formula IV;
and wherein said aryl to aryl coupling is achieved by copolymerization of a composition comprising a phosphorus-containing compound of Formula III and/or Formula IV containing at least two halogen groups with an aryl comonomer containing at least two boronic acid functional groups, a dihydroxy aryl bridging group and/or a diamine aryl bridging group to effect aryl to aryl coupling and produce the phosphite-containing polymer.

The fourth aspect of the present invention provides a polymeric, phosphorus-containing composition made as described in aspect three.

The fifth aspect of the present invention provides a method to produce a polymeric phosphorus-containing composition by:
1. aryl to aryl oxidative coupling of a composition comprising a monomer having the structure: 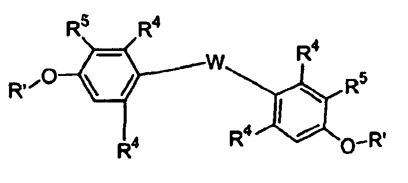 wherein:
   W is C₆-C₂₀ arylene, C₁-C₂₀ alkylene or C₁-C₂₀ cycloalkylene;
   each R' is individually hydrogen or a hydroxyl protective group selected from, but not limited to, alkyl, alkoxyalkyl, carbonylalkyl, or a crown ether formed by taking both R' groups together;
   each R⁴ is independently H, C₁ to C₂₀ branched or straight chain alkyl, C₁ to C₂₀ cycloalkyl, or C₆ to C₂₀ aryl; and,
   each R⁵ is independently C₁ to C₂₀ branched or straight chain alkyl, C₁ to C₂₀ cycloalkyl, or C₆ to C₂₀ aryl and,
2. if R' is a hydroxyl protective group, converting R' to H or alkali metal or alkaline earth metal, and
3. phosphonylating the product of step (1) if R' is other than a hydroxyl protective group, or phosphonylating the product of steps (1) and (2) if R' is a hydroxyl protective group, with at least one diaryloxyphosphite [-P(-O-Ar)₂], diarylphosphine [-P(Ar)₂], and/or aryl,aryloxyphosphinite [-P(Ar)(-O-Ar)], wherein each Ar is individually phenyl, substituted phenyl, naphthyl, or substituted naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide.

The sixth aspect of the present invention provides a polymeric, phosphorus-containing composition made as described above in aspect five.

The seventh aspect of the present invention provides a catalyst comprising at least one of the polymeric, phosphorus-containing compositions made as described above and at least one Group VIII metal.

The eighth aspect of the present invention provides the use of the catalyst composition from aspect seven for a hydrocyanation process comprising reacting an unsaturated organic compound with HCN in the presence of the polymeric, phosphorus-containing composition and the Group VIII metal with or without a Lewis acid.

The ninth aspect of the present invention provides the use of the catalyst composition from aspect seven for an isomerization process comprising reacting an unsaturated organic nitrile compound in the presence of the polymeric, phosphorus-containing bidentate ligand composition and a Group VIII metal.

The tenth aspect of the present invention provides the use of the catalyst composition from aspect seven for a hydroformylation process comprising reacting an unsaturated organic compound with CO and H₂ in the presence of the polymeric, phosphorus-containing bidentate ligand composition and the Group VIII metal.

### DETAILED DESCRIPTION OF THE INVENTION

The phosphorus-containing compositions of the present invention may be viewed as belonging to the family of bidentate ligands, because each pair of trivalent phosphorus atoms is potentially available to simultaneously coordinately bond to a single Group VIII metal atom; i.e., the phosphorus atoms represent electron donors to the same metal atom of the resulting metal complex.

The pair of trivalent phosphorus atoms comprising the bidentate ligand moiety of the polymeric composition is characterized as involving three phosphorus to oxygen bonds (i.e., a phosphite structure), one phosphorus to oxygen bond and two phosphorus to carbon bonds (i.e., a phosphinite structure), or two phosphorus to oxygen bonds and one phosphorus to carbon bond (i.e., a phosphonite structure). In each case, one of the phosphorus to oxygen bonds is associated with the oxygen of the hydroxyl group of the 2,2'-dihydroxyl-1,1'-binaphthalene or 2,2'-dihydroxyl-1,1'-biphenylene structures. The two other bonds associated with the trivalent phosphorus involve a pair of phosphorus to aryl carbon bonds, a pair of phosphorus to aryloxy oxygen bonds, or one phosphorus to aryl carbon bond and one phosphorus to aryloxy oxygen bond. Each aryl (Ar) or aryloxy (-O-Ar) is independently phenyl, naphthyl, substituted phenyl, or substituted naphthyl. For any individual trivalent phosphorus, the pair of aryl or aryloxy may optionally be linked to each other either directly or through a linking unit.

The first aspect of the present invention provides a method to produce polymeric, phosphorus-containing, bidentate ligand compositions by aryl to aryl coupling of the compound of Formula I and/or the compound of Formula II to produce a polymer containing hydroxylated biaryls. Phosphonylation of this polymer will then produce the polymeric, bidentate, phosphorus-containing composition.

The polymeric backbone may be represented conceptually as follows: 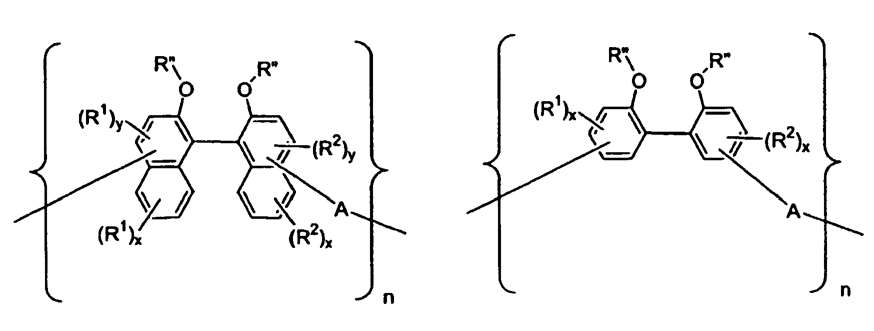 wherein:
n is an arbitrary numerical value representing the average degree of polymerization achieved;
A is either a direct aryl to aryl chemical bond, a bridging aryl unit, or alkylidene;
and wherein R" is hydrogen, a hydroxyl protective group, a diarylphosphonyl, diaryloxyphosphonyl, or aryl,aryloxyphosphonyl;
and R¹ and R² are as previously defined. The values of x and y are less than defined above and are reduced appropriately by the number of A attachments. The polymers are subsequently phosphonylated to produce the polymeric, phosphorus-containing composition.

It should be appreciated that the actual polymeric structure derived from the aryl to aryl coupling reaction according to the present invention may be far more complex than literally illustrated in the above structural representation. For example, the selection of R¹ and R² ring substitution (including, in particular, the number and position of the hydrogens or other functional groups at the aryl sites to be coupled) will influence the polymerization, including polymeric branching as a possibility if multiple coupling occurs at a single aryl. It is further contemplated that the use of a tri-functional comonomer will also produce polymeric branching. The use of mixtures of compounds of Formulas I, II, III and/or IV, that may vary with respect to the R¹ and R², as well as optional use of other comonomers, further complicates the various types of copolymer that may result. As such, the above structural representation of the resulting polymeric backbone should be interpreted appropriately to include such structural variations.

Phosphonylation of this polymer will produce the bidentate, phosphorus-containing composition. Contacting a diaryloxy phosphorochloridite, ClP(-O-Ar)₂, with polymers containing hydroxylated biaryls will give polymers containing bidentate phosphites. The reaction of aryloxy phosphorochloridite [Cl₂P(-O-Ar)] with polymers containing hydroxylated biaryls in the presence of base will lead to polymers containing monodentate phosphites. Preferred bases are organic bases such as trialkylamines. The use of diarylchlorophosphine with polymers containing hydroxylated biaryls in the presence of base will lead to polymers containing bidentate phosphinite units. The use of CIP(Ar)(-OAr) with polymers containing hydroxylated biaryls in the presence of base will lead to polymers containing bidentate phosphonite units. The use of mixtures of Cl₂P(-O-Ar), ClP(-O-Ar)₂, Cl₂P(Ar), and ClP(Ar)(Ar), with polymers containing hydroxylated biaryls will give polymers containing bidentate phosphinite, monodentate phosphinite, monodentate phosphite and bidentate phosphite groups. Similarly, mixtures containing CIP(Ar)(-O-Ar), CIP(-O-Ar)₂, and ClP(Ar)₂ will lead to polymers containing bidentate phosphonite, bidentate phosphite and bidentate phosphinite groups. Other combinations are possible. Each Ar is individually phenyl, substituted phenyl, naphthyl, or substituted naphthyl, provided that the two Ar groups that are directly or indirectly bonded to the same phosphorus atom may be linked to each other by a linking unit selected from the group consisting of direct bond, alkylidene, secondary or tertiary amine, oxygen, sulfide, sulfone, and sulfoxide.

The biphenol derivatives and the binaphthol derivatives of the above illustrated 2,2'-dihydroxyl-1,1'-binaphthalene and 2,2'-dihydroxyl-1,1'-biphenylene structures (i.e., the diols) can be prepared by a variety of routes known in the art and represent monomeric precursors to the desired polymeric, phosphorus-containing bidentate ligand composition of the instant invention. Further details of their synthesis may be found in, for example: Tetrahedron, 1971, 1875; J. Chem. Soc., Perkin Trans. II, 1983, 587; J. Org. Chem., 1984, 49, 4456; J. Org. Chem., 1983, 48, 4948; and Tetrahedron Lett., 1990, 31, 413. Compositions comprising substituted [1, 1'] binaphthalenyl-2,2'-diol and/or substituted biphenyl-2,2'-diol (i.e., 1,1'-bi-2,2'-naphthol and/or 1,1'-bi-2,2'-phenol) may be prepared and polymerized as described by a variety of methods known in the art, such as those illustrated in U. S. Pat. No. 5,889,134 and Chem. Rev., 1998, 98, 2405. For example, poly(1,1'-bi-2,2'-naphthol) can be prepared according to J. Am. Chem. Soc., 1996, 61, 5200, by coupling of protected 6,6'-dibromo-1,1'-bi-2,2'-naphthol catalyzed by nickel(II) chloride and excess zinc.

For purposes of the present invention, it is intended that the structures shown in Formulae I, II, III, and IV, and the expressions "2,2'-dihydroxyl-1,1'-binaphthalene" and "2,2'-dihydroxyl-1,1'-biphenylene" include not only the diol structure but also the corresponding so-called protected diol structures wherein the hydrogen of the hydroxyl group is temporarily replaced by various organic radicals as generally known in the art. Any protected diol structure will need to have the protective group removed and replaced with H or alkali metal or alkaline earth metal prior to phosphonylation.

Also, for purposes of this invention, the term "phosphonylation" means that each of the hydroxyl groups or protected hydroxyl groups of the respective 2,2'-dihydroxyl-1,1'-binaphthalene or 2,2'-dihydroxyl-1,1'-biphenylene structures are replaced with a trifunctional phosphorus. Typically (after removal of the protective groups by hydrolysis or the like), the hydroxyl groups are reacted with at least one diaryloxychlorophosphonite [ClP(-O-Ar)₂], diarylchlorophosphine [ClP(Ar)₂], and/or aryl,aryloxychlorophosphinite [ClP(Ar)(-O-Ar)], producing a phosphorus to oxygen chemical bond with the elimination of hydrogen chloride or an equivalent. Treatment of the alkoxide with a diaryloxychlorophosphonite [ClP(-O-Ar)₂], a diarylchlorophosphine [ClP(Ar)₂], or an aryl,aryloxychlorophosphinite [ClP(Ar)(-O-Ar)], or mixture thereof produces a phosphorus to oxygen chemical bond with the elimination of metal chloride or an equivalent.

Preferably, the phosphonylation reaction is performed with at least two equivalents of the diarylchlorophosphonite [ClP(Ar)₂], diaryloxychlorophosphonite [ClP(-O-Ar)₂], arylaryloxychlorophosphinite [ClP(Ar)(-O-Ar)] or mixture thereof relative to the diol structure. Advantageously, a stoichiometric excess of a trialkylamine or the like is present during phosphonylation to drive the reaction by salt formation with hydrogen chloride being inherently co-produced. The aryl (Ar) is individually phenyl, naphthyl, substituted phenyl, or substituted naphthyl with the proviso that for any individual phosphorus the pair of aryls or aryloxys or combination aryl and aryloxy may optionally be linked to each other either directly or through a linking unit. When the aryl is a substituted phenyl or substituted naphthyl, the substitution involves a radical or radicals selected from C₁ to C₂₀ branched or straight chain alkyl, C₁ to C₂₀ cycloalkyl, acetal, ketal, C₆ to C₂₀ aryl, F, Cl, Br, CN, perhaloalkyl, -CHO, -OR³, -C(O)R³, -CO₂R³, -S(O)R³, -SO₂R³, -SO₃R³, and cyclic ether; where each R³ is independently C₁ to C₂₀ branched or straight chain alkyl, C₁ to C₂₀ cycloalkyl, or C₆ to C₂₀ aryl. In a preferred embodiment, one of the substitutents ortho to the oxygen in the O-Ar group is hydrogen and the other ortho substituent is C₁ to C₂₀ secondary or straight chain alkyl, C₁ to C₂₀ cycloalkyl, acetal, ketal, C₆ to C₂₀ aryl, OR³ or cyclic ether.

Preferably, each Ar is independently selected from the group depicted below: 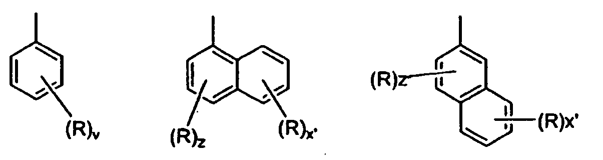 wherein v = 0 to 5; z = 0 to 3, and x' = 0 to 4.

The aryl groups associated with the phosphorus of a diaryloxyphosphonite [-P(-O-Ar)₂], or a diarylphosphine [-P(Ar)₂], or aryl,aryloxyphosphinite [-P(Ar)(-O-Ar)], may be linked to each other either directly through an aryl carbon to aryl carbon bond or through a linking group, which may be -C(R⁴)(R⁴)-, -O-, -N(R⁴)-, -S-, -S(O)₂-, and -S(O)-; where each R⁴ is independently H, C₁ to C₂₀ branched or straight chain alkyl, C₁ to C₂₀ cycloalkyl, or C₆ to C₂₀ aryl.

Bidentate phosphites (compounds of Formula III or of Formula IV having three phosphorus to oxygen bonds for each phosphorus, a = 2 and b = 0 in Formula III or Formula IV) can be prepared, as described in U. S. Pat. No. 5,235,113, by contacting phosphorochloridites with a binaphthol. Alternatively, the phonsphonylated, substituted 2,2'-dihydroxyl-1,1'-binaphthalene or 2,2'-dihydroxyl-1,1'-biphenylene may be prepared as in U. S. Pat. Nos. 6,031,120 and 6,069,267 where the phosphorochloridite is prepared, *in situ*, from phosphorus trichloride and a phenol such as o-cresol and then treated in the same reaction vessel with an aromatic diol to give the bidentate phosphite.

Alternatively, the phosphonylation reaction can be carried out as described in U. S. Pat. No. 5,910,600. The first step is to convert the -OH groups of the substituted 2,2'-dihydroxyl-1,1'-binaphthalene or 2,2'-dihydroxyl-1,1'-biphenylene to -OM groups wherein M is an alkali metal or an alkaline earth metal, followed by treatment with at least one of diaryloxychlorophosphonite [ClP(-O-Ar)₂], diarylchlorophosphine [ClP(Ar)₂], and/or aryl,aryloxychlorophosphinite [CIP(Ar)(-O-Ar)], producing a phosphorus to oxygen chemical bond with the elimination of metal chloride or an equivalent.

Phosphorochloridite may be prepared by a variety of methods known in the art, as described in, for example, Polymer, 1992, 33, 161; Inorganic Synthesis 1966, 8, 68; U. S. Pat. No. 5,210,260; and Z. Anorg. Allg. Chem., 1986, 535, 221. Also, phosphorochloridites of 1-naphthols can be prepared *in situ* from PCl₃ and 1-naphthol in the presence of a base like trialkylamine. Use of substituted 1,1'-bi-2-phenol or substituted 1,1'-bi-2-naphthol with PCl₃ will lead to phosphorochloridite, ClP(-O-Ar)₂, wherein the two Ar groups are linked directly together. The two Ar groups that are directly or indirectly bonded to the same phosphorus atom may be linked to each other by a linking unit selected from direct bond, alkylidene, secondary or tertiary amine, oxygen, sulfide, sulfone, and sulfoxide. Another process for preparing the phosphorochloridite comprises treatment of N,N-dialkyl diarylphosphoramidite with HCl. ClP(OMe)₂ has been prepared in this manner, see Z. Naturforsch, 1972, 27B, 1429. Phosphorochloridites derived from substituted phenols have been prepared using this procedure as described in commonly assigned U. S. Pat. No. 5,821,378, incorporated herein by reference.

The reaction of phosphorus trichloride with one equivalent of substituted phenol will lead to Cl₂P(-O-Ar), aryloxy phosphorodichloridite. The Cl₂P(-O-Ar) can also be prepared from [(alkyl)₂N]₂PCl with a substituted phenol followed by treatment with HCl. The reaction of aryloxy phosphorochloridite [Cl₂P(-O-Ar)] with a substituted biphenol or substituted binaphthol in the presence of base will lead to a monodentate phosphite. Preferred bases are organic bases such as trialkylamines.

Bidentate phosphinite compounds (i.e., those compounds of Formula III or Formula IV having one phosphorus to oxygen bond and two phosphorus to carbon bonds, with a = 0 and b = 2) may be synthesized by reaction of diarylchlorophosphine with the present diol structures (i.e., 2,2'-dihydroxyl-1,1'-binaphthalene and/or 2,2'-dihydroxyl-1,1'-biphenylene) in the presence of base. Preferably, the base is a trialkylamine. See, for example, U. S. Pat. No. 5,523,453, incorporated herein by reference. More preferably, the trialkylamine is one with C₁ to C₁₂ branched or straight chain alkyl groups. Most preferred is triethylamine.

Bidentate phosphonite compounds (i.e., those compounds of Formula III or Formula IV having two phosphorus to oxygen bonds and one phosphorus to carbon bond, with a = 1 and b = 1) may be sythesized by reaction of the CIP(Ar)(-O-Ar) with the present diol structures (i.e., 2,2'-dihydroxyl-1,1'-binaphthalene and/or 2,2'-dihydroxyl-1,1'-biphenylene) in the presence of base. Preferably the base is a trialkyamine.

Examples of phosphorochloridites useful for phosphonylation include, but are not limited to, those shown below:

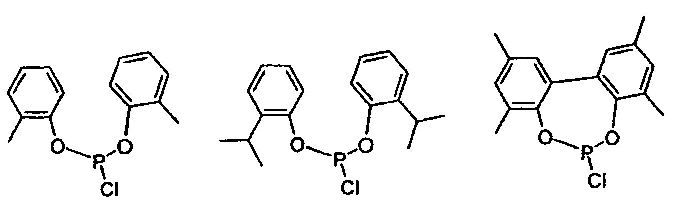

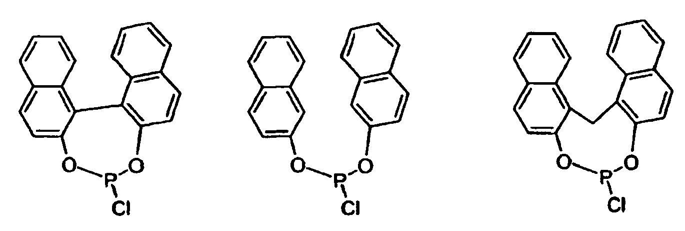

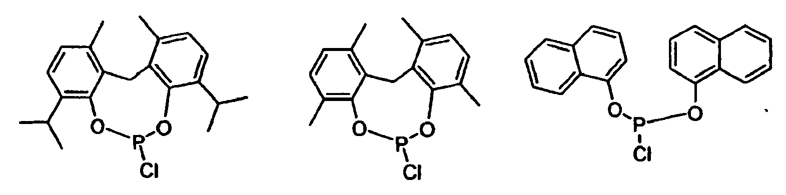

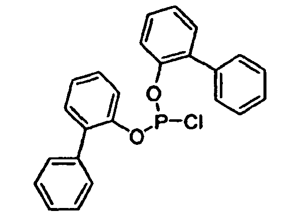

The aryl to aryl coupling reactions of the first aspect of the present invention may be categorized as follows:
(i) polymerization by direct oxidative coupling between aryl groups of the compounds of Formula I and/or Formula II,
(ii) copolymerization by coupling of the aryl group of the compounds of Formula I and/or Formula II with an aryl comonomer,
(iii) copolymerization by coupling of the compounds of Formula I and/or Formula II by a Friedel-Crafts type reaction in the presence of dialkylating or dibenzylating or diacylating comonomer, or
(iv) copolymerization of the compound of Formula II with an aldehyde.

The compound of Formula III and/or the compound of Formula IV may be used as comonomer in processes described in (ii) and (iii) above.

### i. Polymerization via direct oxidative coupling:

Polymers containing 1,1'-bi-2-naphthol and 1,1'-bi-2-phenol groups (i.e., aryl to aryl coupled 2,2'-dihydroxyl-1,1'-binaphthalene and 2,2'-dihydroxyl-1,1'-biphenylene structures) may be prepared by a variety of methods known in the art. For example, oxidative coupling of phenols is a fundamental method for the synthesis of hydroxylated biaryls, as described in "Oxidative Coupling of Phenols", Ed.: W.I. Taylor, and A.R. Battersby, New York: M. Dekker Inc., 1967; Organic Preparations and Procedures Int., 1975, 7, 255; J. Org. Chem., 1963, 28, 1063; J. Org. Chem., 1968, 34, 2388; J. Org. Chem., 1984, 49, 4456; J. Org. Chem., 1983, 48, 4948; J. Org. Chem., 1980, 45, 749; J. Org. Chem., 1981, 46, 4545; Tetrahedron Lett., 1977, 4447; Tetrahedron, 1992, 43, 9483; Tetrahedron, 1992, 48, 9483; and Photochemistry, 1988, 27, 3008.

In particular, J. Org. Chem., 1968, 34, 2388, describes the oxidative coupling of phenols using vanadium tetrachloride and/or vanadium oxytetrachloride to produce the desired biphenol structure without hydroxyl group protection. Thus, direct oxidative coupling can be used as shown to prepare the starting biphenol and/or binaphthol and, in principle, can be used as well in the subsequent polymerization and/or copolymerization of the same.

In comparison, J. Am. Chem. Soc., 1996, 61, 5200 describes direct coupling of methyl ether protected 6,6'-dibromo-1,1'-bi-2,2'-naphthol catalyzed by nickel(II) chloride and excess zinc to prepare poly(1,1'-bi-2,2'-naphthol). This polymerization reaction can be carried out at temperatures between 40 to 120°C and, in principle, can be used as well in coupling a protected phenol and/or naphthol to produce a protected biphenol and/or protected binaphthol. The protecting group is then removed to yield the corresponding diol structure.

### ii. Copolymerization with aryl comonomer:

Copolymers containing 1,1'-bi-2-naphthol and 1,1'-bi-2-phenol groups (i.e., aryl to aryl coupled 2,2'-dihydroxyl-1,1'-binaphthalene and 2,2'-dihydroxyl-1,1'-biphenylene structures) in combination with one or more aryl comonomers may be prepared by a variety of methods known in the art for oxidative coupling including the above described methods. In such copolymerizations, the aryl comonomer becomes the Ar' bridging aryl unit between the 1,1'-bi-2-naphthol and 1,1'-bi-2-phenol groups of the resulting copolymer.

One method for making a copolymer poly(binaphthol) or copolymer poly(biphenol) involves first halogenating the 2,2'-dihydroxyl-1,1'-binaphthalene and/or 2,2'-dihydroxyl-1,1'-biphenylene structures at the sites at which the polymer is to be linked (i.e., the at least one R¹ and the at least one R² intended to represent aryl to aryl polymeric coupling). Following halogenation, protecting groups are provided on any naphtholic or phenolic hydroxyl groups. Suitable protecting groups include, but are not limited to, ethers, alkyls, esters, and crown ethers. Other protecting groups as generally known to those skilled in the art of protecting hydroxyl moieties during reactions are suitable for this purpose. The protected and halogenated binaphthol and/or biphenol can then be polymerized in the presence of a nickel(0) or nickel(II) catalyst, as described in J. Org. Chem., 1996, 61, 5200. Suitable nickel catalysts include NiCl₂ or (1,5-cyclooctadiene)₂Ni. A polymerization reaction catalyzed by NiCl₂ typically requires the presence of excess zinc. The average molecular weight of the resulting polymer can be controlled to some extent by the amount of NiCl₂ catalyst used in the reaction. After polymerization, the hydroxyl protecting groups are removed to give the poly(binaphthol) or poly(biphenol).

The above described, protected and halogenated binaphthol and/or biphenol monomers can be copolymerized with an aryl diamine comonomer in the presence of a Group VIII transition metal catalyst such as palladium or nickel. Preferably the Group VIII transition metal is palladium and the catalyst is Pd(OAc)₂. The polymerization can be performed by reacting the aryl halide monomer with the aryl amine comonomer using typical Pd-catalyzed carbon-nitrogen coupling procedures, such as those described in J. Am. Chem. Soc., 1998, 120, 4900 and J. Org. Chem., 2000, 65, 1144. The polymerization can be done under refluxing conditions using high boiling solvents (i.e., toluene), a base (i.e., K₂CO₃) and a phosphine ligand (i.e., 2,2'-bis(diphenylphosphino)-1,1'-binaphthyl).

Examples of such aryl, diamine comonomer compounds include, but are not limited to those shown below:

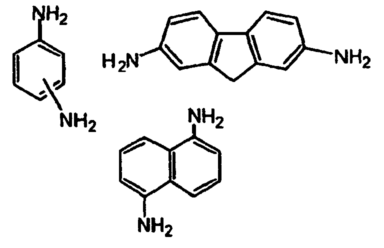

The desired poly(binaphthol) or poly(biphenol) may also be formed by a Suzuki coupling reaction, as described in Synth. Commun., 1981, 11, 513; J. Am. Chem. Soc., 1991, 113, 7411; Acc. Chem. Res., 1982, 15, 179; J. Org. Chem., 1998, 63, 7536; Tetrahedron Lett., 1998, 29, 2933; Tetrahedron Lett., 1998, 29, 2937; Angew. Chem. Int. Engl. Ed., 1999, 38, 2345; Monatsch. Chem., 1998, 1319; U. S. Pat. No. 5,889,134; and Chem. Rev., 1998, 98, 2405. In such a case, the protected and halogenated binaphthol and/or biphenol monomers and an aryl comonomer with boronic acid or ester functional groups at the linkage sites are copolymerized. As such, the aryl comonomer with boronic acid functional groups becomes the bridging aryl unit, Ar', coupling the 2,2'-dihydroxyl-1,1'-binaphthalene and/or 2,2'-dihydroxyl-1,1'-biphenylene structures. The polymerization can be performed under refluxing conditions using high boiling solvents (i.e., toluene or 1,4-dioxane) and a base (i.e., K₂CO₃). An effective Group VIII transition metal catalyst is Pd(PPh₃)₄. The hydroxyl protective group, R', can then be removed to produce polymeric biphenol or polymeric binaphthol. Methods to accomplish this reaction are found in "Protective Groups in Organic Synthesis", T. W. Greene and P. G. M. Wuts, New York: Wiley-Interscience, 1999. For example, when R' = Me, the replacement with H can be accomplished by reaction with BBr₃ at low temperature (-30°C). The completion of this replacement can be ascertained by ¹H NMR (disappearance of OMe resonance) or by IR (appearance of OH stretch).

Specifically, an example of this synthetic route is shown below: 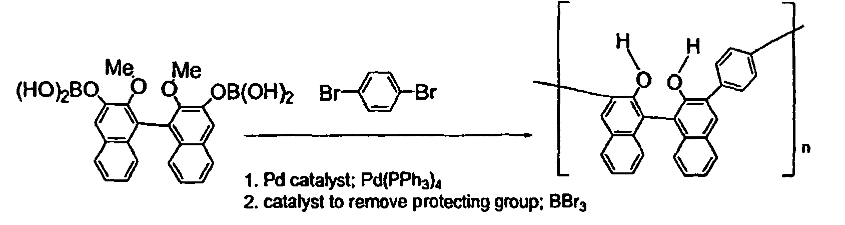 where n is an arbitrary number representing the average degree of polymerization obtained.

Alternatively, one can start with the bromo groups on the protected binaphthol and the boronic acid groups on the benzene ring. Polymers that have a structure containing different biphenyl or binaphthyl monomer units can be obtained by coupling a mixture of aryl[bromide]ₘ reagents with a mixture of aryl[boronic]ₘ acids wherein m = 2 to 6, preferably m = 2 to 3.

Examples of the aryl comonomer with boronic acid functional groups useful in the present invention include, but are not limited to, those shown below: 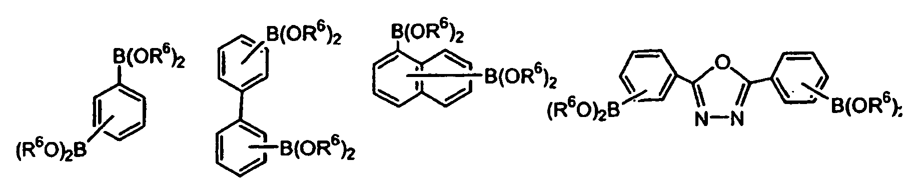 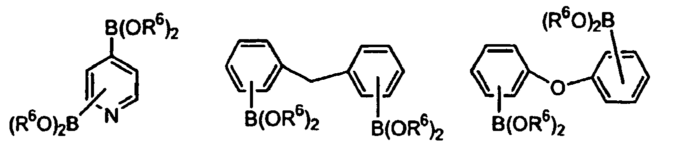 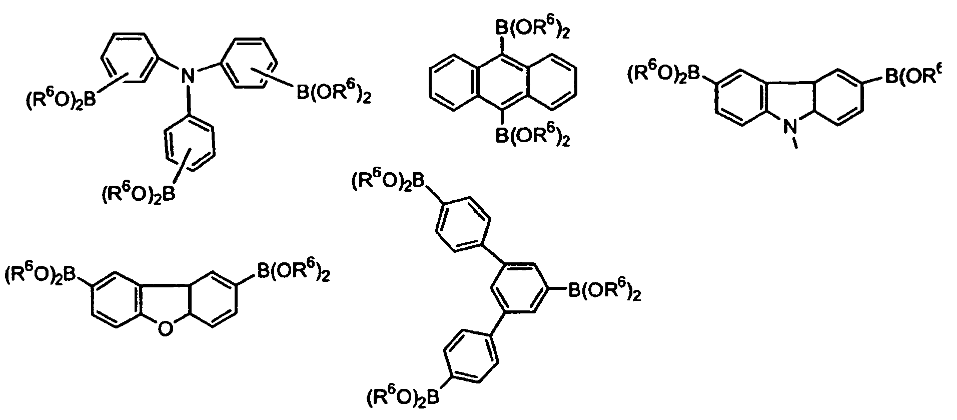 wherein each R⁶ is independently selected from the group consisting of H, C₁ to C₂₀ branched or straight chain alkyl, and C₁ to C₂₀ cycloalkyl. Optionally, two R⁶ groups may be linked by a C₁ to C₂₀ alkyl or cycloalkyl group. Preferably, each R⁶ is independently H or C₁ to C₂₀ straight chain alkyl.

Other halogenated aryl comonomers can be used in the polymerization step and, in principle, represent a diluent relative to the protected and halogenated binaphthol and/or biphenol. Examples of comonomers include, but are not limited to, those shown below where X represents the halogen.

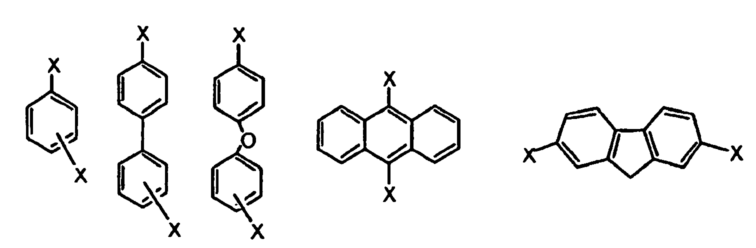

Alternatively, the protected and halogenated binaphthol and/or biphenol is reacted with magnesium followed by a trialkylborate and then hydrolyzed to obtain a diboronic acid-substituted binaphthol and/or biphenol, as described in Macromolecules, 1996, 29, 1082 and Macromolecules, 1996, 29, 5075. The resulting diboronated and protected binaphthol and/or biphenol are then copolymerized with a dihalogenated aryl bridging group by the Suzuki coupling reaction. Preferably, a palladium catalyst and a triarylphoshine ligand are employed. In essence, the roles of the dihalogenated and diboronated comonomers are interchanged relative to the Suzuki coupling described above.

Diboronated and protected binaphthol and/or biphenol compounds useful in the present invention can also be prepared by ortho-lithiation of the biphenol and/or binaphthol precursors followed by reaction with B(OEt)₃, as described in J. Org. Chem., 1998, 63, 7536. Alternatively, these compounds can be synthesized by starting with the bromide precursors which are lithiated and quenched with B(OEt)₃, as described in Macromolecules, 1996, 29, 1082. Again, other comonomers as previously described can be used in the preparation of the boronated comonomer, and can essentially be viewed as comonomer diluents of the protected binaphthol and/or biphenol comonomer. Also, mixtures of the protected binaphthol and/or biphenol can be used as comonomer starting reactant.

The protected and boronated binaphthol and/or biphenol comonomer reactants and mixtures thereof may also be coupled by copolymerization with a dihydroxy aryl bridging group and/or a diamine aryl bridging group (i.e., a biphenol or bianiline bridging group). Typically, a Group VIII transition metal such as palladium or copper is employed as catalyst. Preferably the catalyst is Cu(OAc)₂. The polymerization of the dihydroxy aryl or diamine aryl bridging group can be performed following procedures described in Tetrahedron Lett., 1998, 39, 2933 and Tetrahedron Lett., 1998, 39, 2937, using Cu(OAc)₂ and Et₃N at room temperature. The polymerization of the diamine aryl bridging group may be performed as described in Acc. Chem. Res., 1998, 31, 805.; Angew. Chem. Int. Ed. Engl., 1995, 34, 1348.; and J. Amer. Chem. Soc., 1998, 120, 4900. Examples of dihydroxy aryl bridging group and/or diamine aryl bridging group comonomers include, but are not limited to, those shown below:

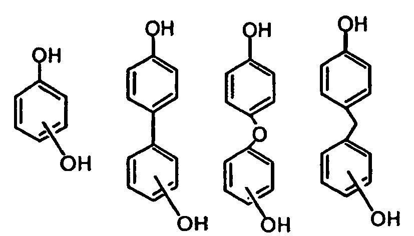

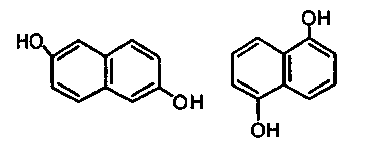

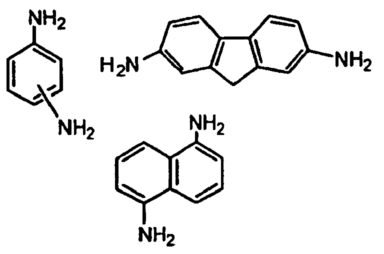

### iii. Copolymerization via Friedel-Crafts type reaction

When the aryl to aryl coupling reaction according to the instant invention involves copolymerization by coupling of the 2,2'-dihydroxyl-1,1'-binaphthalene or 2,2'-dihydroxyl-1,1'-biphenylene structure using a Friedel-Crafts type reaction in the presence of dialkylating or dibenzylating or diacylating comonomer, the reaction is performed in the presence of a Lewis acid. The dialkylating comonomer is typically a dihalogenated or terminal diene organic bridging unit; however a mixed terminal olefin and halogen substituted organic bridging unit may also be used. Similarly, the diacylating comonomer may be a dicarboxoyl halide substituted organic compound. Although the Friedel-Crafts type reaction is amenable to the use of branched and/or linear dialkylating and diacylating agents, preferably the bridging unit (comonomer) is an aromatic or substituted aromatic ring structure of six to forty carbons and includes heterocyclic and bridged aryl structures as previously described herein. Preferably, the dialkylating monomer is a compound containing at least two benzyl halide groups to achieve aryl to aryl coupling.

The alkylation and benzylation reactions of phenols are known organic synthetic methodologies to make substituted phenols; for example, Friedel-Crafts alkylation of phenols is described in "Advanced Organic Chemistry", 4th edition, Ed.: J. March, p. 536, 1992, as well as in other references: U. S. Pat. Nos. 1,972,599; 2,726,270; and 2,784,239; J. Amer. Chem. Soc., 1992,114,4067-4079; J. Amer. Chem. Soc., 1950, 72, 4171; J. Amer. Chem. Soc., 1930, 52, 4484; J. Chem. Soc. Perkin Trans. II, 1982, 1193-1198; Chem. Ber., 1894, 27, 1615; Monatsh. Chem., 1929, 53/54, 736; J. Org. Chem., 1950, 396; Bull. Chem. Soc. Jpn., 1985, 58, 115-119; and Bull. Soc. Chim. Fr., 1935, 497, 513.

The use of monomers containing three or more benzyl chloride groups with biphenols containing more than two hydrogen as R groups leads to a highly crosslinked polymer. Polyfunctional olefins, alcohols, acid halides, and alkyl halides can be used instead of benzyl halides. Examples of comonomers useful in the present invention include, but are not limited to: 1,4-divinylbenzene; 2,5-dimethyl-2,5-hexanediol; 2,5-dimethyl-1,5-hexadiene; 2,5-dichloro-2,5-dimethylhexane; 4,4'-bis(chloromethyl)-1,1'-biphenyl; 2,2'-bis(bromomethyl)-1,1'-biphenyl; α,α'-dichloro-*p*-xylene; α,α'-dichloro-*m*-xylene; and vinyl benzyl chloride. Typical Lewis acid catalysts useful for the polymerization step include, for example, zinc chloride and aluminum chloride.

### iv. Copolymerization with an aldehyde

When the aryl to aryl coupling reaction according to the instant invention involves copolymerization of the aryl group of the compound of Formula II with an aldehyde, the 1,1'-bi-2-phenol (i.e., aryl to aryl coupled 2,2'-dihydroxyl-1,1'-biphenylene structures) is reacted with at least one aldehyde of the formula R⁷C(O)H in the presence of an acid or base catalyst. R⁷ is H, C₁ to C₂₀ branched or straight chain alkyl, C₁ to C₂₀ cycloalkyl, or C₆ to C₂₀ aryl. In this case, the aryl to aryl coupling will be through an alkylidene diradical derived from the aldehyde. The polymeric, phosphorus-containing composition is a phenolic resin derived from the reaction of substituted biphenols with aldehydes. A review of phenolic resin can be found in "Comprehensive Polymer Chemistry: The Synthesis, Characterization, Reactions & Applications of Polymers", Ed.: G. Allen et *al*., Vol. 5, 1989. Starting biphenols substituted in the 3,3' positions are preferred. Preferred examples are 3,3'-dimethyl-2,2'-biphenol; 3,3'-di-n-propyl-2,2'-biphenol; 3,3'-di-isopropyl-2,2'-biphenol; 3,3',6,6'-tetramethyl-2,2'-biphenol; 3,3'-di-n-propyl-6,6'-dimethyl-2,2'-biphenol; 3,3'-di-isopropyl-6,6'-dimethyl-2,2'-biphenol.

Substituted biphenols can be prepared by oxidative coupling of phenols. An example is the preparation of 3,3',6,6'-tetraalkyl-2,2'-biphenols. 2,5-Dialkylphenol can be chlorinated at the para position to give 2,5-dialkyl-4-chlorophenol. The 2,5-dialkyl-4-chlorophenol can be oxidatively coupled to give the dimeric chlorophenols. The Cl atoms can be removed by hydrogenolysis to provide the required 3,3',6,6'-tetraalkyl-2,2'-biphenols. Alternatively, the para position of 2,5-dialkylphenol can be protected with tert-alkyl groups. Dimerization of 2,5-dialkyl-4-*tert*-alkylphenol by oxidative coupling leads to biphenol containing *tert*-alkyl groups in the 5,5'-positions. Dealkylation leads to the required 3,3',6,6'-tetraalkyl-2,2'-biphenols.

The third aspect of the present invention provides a method to produce a polymeric, phosphorus-containing composition by copolymerization of a composition comprising at least one substituted phosphonylated 2,2'-dihydroxyl-1,1'-binaphthalene as shown in Formula III and/or at least one substituted phosphonylated 2,2'-dihydroxyl-1,1'-biphenylene as shown in Formula IV with an aryl comonomer containing at least two boronic acid functional groups, a dihydroxy aryl bridging group and/or a diamine aryl bridging group to effect aryl to aryl coupling and produce the phosphite-containing polymer.

Preferably R¹ and R² are independently H, C₁ to C₂₀ branched or straight chain alkyl, C₁ to C₂₀ cycloalkyl, C₁ to C₂₀ acetal, C₁ to C₂₀ ketal, C₆ to C₂₀ aryl, OR³, CO₂R³, F, Cl, Br, SO₃R³, CN, C₁ to C₂₀ perhaloalkyl, S(O)R³, SO₂R³, CHO, C(O)R³, B(OR³)₂, NR³₂, or C₁ to C₂₀ cyclic ether. Most preferably, R¹ and R² are lower alkyls such as methyl, ethyl, *n*-propyl, 2-propyl, n-butyl, 2-butyl, t-butyl groups or the like and are selectively positioned on the respective aryl groups such as to enhance or influence the relative position of the R¹ and R² that are reserved for and/or involved in aryl to aryl coupling (e.g., on the biphenol, typically para to the hydroxyl group). For aryl to aryl coupling, the preferred R¹ and R² are halogen groups, such as bromine. Each R³ is independently C₁ to C₂₀ branched or straight chain alkyl, C₁ to C₂₀ cycloalkyl, or C₆ to C₂₀ aryl.

Typically, the 2,2'-dihydroxyl-1,1'-binaphthalene or a 2,2'-dihydroxyl-1,1'-biphenylene structure is phosphonylated with diarylchlorophosphonite [ClP(Ar)₂], diaryloxychlorophosphonite [CIP(-OAr)₂], aryl,aryloxychlorophosphinite [CIP(Ar)(-O-Ar)] or the like to produce the phosphonylated, substituted 2,2'-dihydroxyl-1,1'-binaphthalene or a 2,2'-dihydroxyl-1,1'-biphenylene.

In the fifth aspect, the present invention provides a method to prepare a polymeric, phosphorus-containing composition by:
(1) aryl to aryl oxidative coupling of monomer having the structure: 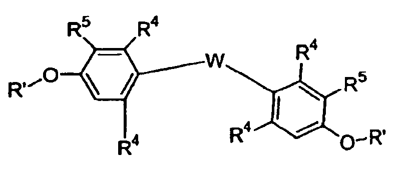 wherein:
   W is C₆ to C₂₀ arylene, C₁ to C₂₀ alkylene or cycloalkylene;
   each R' is individually hydrogen or a hydroxyl protective group selected from, but not limited to, alkyl, alkoxyalkyl, carbonylalkyl, or a crown ether formed by taking both R' groups together;
   each R⁴ is independently H, C₁ to C₂₀ branched or straight chain alkyl, C₁ to C₂₀ cycloalkyl, or C₆ to C₂₀ aryl; and
   each R⁵ is independently C₁ to C₂₀ branched or straight chain alkyl, C₁ to C₂₀ cycloalkyl, or C₆ to C₂₀ aryl; and
(2) phosphonylating with a substituent consisting of at least one diaryloxyphosphite [-P(-O-Ar)₂], diarylphosphine [-P(Ar)₂], and/or aryl,aryloxyphosphinite [-P(Ar)(-O-Ar)], where each Ar is individually phenyl, substituted phenyl, naphthyl, or substituted naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide.

The W of the starting material is C₆-C₂₀ arylene, C₁ to C₂₀ alkylene or cycloalkylene. Examples of the monomer include, but are not limited to, those shown below.

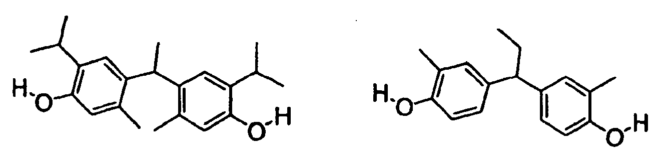

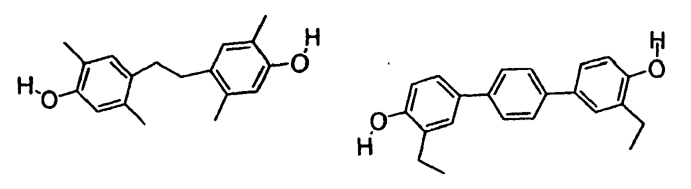

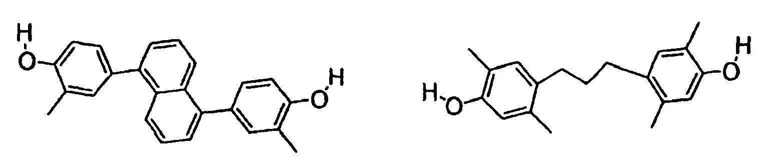

Oxidative coupling of phenols is a fundamental method for the synthesis of hydroxylated biaryls, as described in, for example: "Oxidative Coupling of Phenols", Ed.: W. I. Taylor, and A. R. Battersby, New York: M. Dekker Inc., 1967; Tetrahedron, 1992, 43, 9483; Organic Preparations and Procedures Int., 1975, 7, 255; J. Org. Chem., 1963, 28, 1063; J. Org. Chem., 1968, 34, 2388; J. Org. Chem., 1984, 49, 4456; J. Org. Chem., 1983, 48, 4948; J. Org. Chem., 1980, 45, 749; J. Org. Chem., 1981, 46, 4545; Tetrahedron Lett., 1977,4447; Tetrahedron, 1992, 48, 9483; Photochemistry, 1988, 27, 3008. Coupling of compounds containing two phenol groups linked by a bridging group can lead to polymeric hydroxylated biaryls. An example is oxidative coupling of 4,4'-ethylidenebis(2-isopropyl-5-methylphenol). The starting material, 4,4'-ethylidenebis(2-isopropyl-5-methylphenol), was prepared using a procedure described in Bull. Chem. Soc. Jpn., 1989, 62, 3603, with the W group being -C(CH₃)(H)-. The pair of hydroxyl groups associated with the resultant 2,2'-dihydroxyl-1,1'-biphenylene structure is phosphonylated to produce a polymeric, phosphorus-containing, bidentate ligand composition.

The above processes describe the synthesis of polymeric, phosphorus-containing ligands of various structures. It is known in the art that the solubility properties of polymers are affected by their structure. It is preferred that the polymeric ligands of this invention be as insoluble as resulting polymers are insoluble, they can be separated by filtration from the reaction mixtures in which they are used and then recycled. If the polymeric ligands are partially soluble in the reaction mixture, they may be separated by filtration of the insoluble ligand and then precipitation of the soluble ligand with a solvent in which the ligand has extremely low solubility or by precipitation of the soluble ligand and filtration of the reaction mixture. If the ligands are completely soluble in the reaction mixture, they may be separated by precipitation with a solvent in which the ligand has extremely low solubility.

### Use of Polymeric, Phosphorus-Containing

### Ligands in Catalyst Compositions

The seventh aspect of the present invention provides a catalyst composition comprising at least one of the polymeric ligand compositions of the present invention combined with a Group VIII transition metal, transition metal compound, transition metal complex, or combinations thereof and, optionally a Lewis Acid. Generally, any Group VIII metal or metal compound can be used to combine with the composition. The term "Group VIII" refers to the ACS version of the Periodic Table of the Elements, "CRC Handbook of Chemistry and Physics", 67th edition, Boca Raton, Florida: CRC Press, 1986-1987.

Generally, a Group VIII metal or compound thereof is combined with at least one polymeric ligand of the present invention to provide the catalyst. Among the Group VIII metal compounds, nickel, cobalt, and palladium compounds are preferred for hydrocyanation catalysts. A nickel compound is more preferred. A zero-valent nickel compound containing a ligand that can be displaced by the polymeric ligand of the present invention is the most preferred source of Group VIII metal or Group VIII metal compound. Zero-valent nickel compounds can be prepared or generated according to methods known in the art, such as those described in U. S. Pat. Nos. 3,496,217; 3,631,191; 3,846,461; 3,847,959 and 3,903,120, incorporated herein by reference. Three preferred zero-valent nickel compounds are Ni(COD)₂ (COD is 1,5-cyclooctadiene), Ni(P(O-o-C₆H₄CH₃)₃)₃ and Ni{P(O-*o*-C₆H₄CH₃)₃}₂(C₂H₄), as known in the art.

Alternatively, divalent nickel compounds can be combined with a reducing agent to serve as a source of zero-valent nickel in the reaction. Suitable divalent nickel compounds include compounds of the formula NiZ²₂ where Z² is halide, carboxylate, or acetylacetonate. Suitable reducing agents include metal borohydrides, metal aluminum hydrides, metal alkyls, Li, Na, K, or H₂. Elemental nickel, preferably nickel powder, when combined with a halogenated catalyst, as described in U. S. Pat. No. 3,903,120 (incorporated herein by reference) is also a suitable source of zero-valent nickel.

The chelating arrangement of donor atoms in bidentate ligands results in a strong ligand-metal interaction and thus greatly minimizes the potential for metal leaching. It is possible to alter the spacing between the chelating atoms, the steric environment of these atoms, and the electronic properties of the donor atoms, offering control of ligand coordination properties thereby optimizing catalyst performance.

### Hydrocyanation using Polymeric, Phosphorus-Containing Ligands

In the eighth aspect of the present invention, at least one of the polymeric ligand compositions of the present invention may be used to form a catalyst (with or without a Lewis acid) which may be used for the hydrocyanation of organic compounds. The process comprises contacting, in the presence of the catalyst, an unsaturated organic compound with a hydrogen cyanide-containing fluid under conditions sufficient to produce a nitrile, wherein the catalyst comprises a Group VIII metal, at least one of the polymeric ligands described above, and optionally a Lewis acid. The term "fluid" may be gas, liquid, or both. Any fluid containing about 1 to 100 % HCN can be used. Preferably, the HCN contains less than 10 ppm CO, less than 20 ppm cyanogen, less than 10 ppm epoxide, less than 20 ppm acrylonitrile, less than 20 ppm sulfur dioxide, less than 40 ppm sulfuric acid, and less than 100 ppm peroxides. Pure hydrogen cyanide may be used.

The hydrocyanation process may be carried out, for example, by charging a suitable vessel such as a reactor with an unsaturated compound, catalyst composition, and solvent, if any, to form a reaction mixture. Hydrogen cyanide can be initially combined with other components to form the mixture. However, it is preferred that HCN be added slowly to the mixture after the other components have been combined. Hydrogen cyanide may be delivered as a liquid or as a vapor to the reaction. As an alternative, a cyanohydrin may be used as the source of HCN, as in, for example, U.S. Pat. No. 3,655,723, incorporated herein by reference.

Another suitable technique is to charge the vessel with the catalyst and the solvent (if any) to be used, and feed both the unsaturated compound and the HCN slowly to the reaction mixture.

The molar ratio of unsaturated compound to catalyst can be varied from about 10:1 to about 100,000:1. The molar ratio of HCN to catalyst generally is varied from about 10:1 to 100,000:1, preferably 100:1 to 5,000:1, for a batch operation. In a continuous operation, such as when using a fixed bed catalyst type of operation, a higher proportion of catalyst can be used such as 5:1 to 100,000:1, preferably 100:1 to 5,000:1, HCN to catalyst.

Preferably, the reaction mixture is agitated; for example, by stirring or shaking. The reaction may be run either batchwise or continuously. The reaction product may be recovered by conventional techniques such as distillation.

The hydrocyanation may be carried out with or without a solvent. The solvent, if used, may be liquid at the reaction temperature and pressure and inert towards the olefin and the catalyst. Suitable solvents include hydrocarbons such as benzene, xylene, or combinations thereof; ethers, such as tetrahydrofuran (THF); nitriles, such as acetonitrile, benzonitrile, or adiponitrile, or combinations of two or more thereof. The unsaturated compound to be hydrocyanated may itself serve as the solvent. Hydrocyanation may also be carried out in the gas phase.

The exact temperature is dependent to a certain extent on the particular catalyst being used, the particular unsaturated compound being used and the desired reaction rate. Normally, temperatures of from -25°C to 200°C may be used, the range of 0°C to 150°C being preferred.

Atmospheric pressure is satisfactory for carrying out the reaction, and pressures of from about 0.05 to 10 atmospheres (50.6 to 1013 kPa) are preferred. Higher pressures, up to 10,000 kPa or more, may be used, if desired, but any benefit that may be obtained thereby would need to justify the increased cost of such operations.

The time required may be in the range of from a few seconds to many hours (such as 2 seconds to 24 hours), depending on the particular conditions and method of operation.

The unsaturated compound has 2 to about 30 carbon atoms per molecule and can be cyclic or acyclic. It can have the formula of R⁸CH=CH-CH=CR⁹, CH=CH-(CH₂)_{q}-R¹⁰, CH₃-(CH₂)ₙ- CH=CH-(CH₂)_{q}-R¹⁰, and combinations of two or more thereof in which R⁸ and R⁹ are each independently H, C₁ to C₃ alkyl, or combinations thereof; R¹⁰ is H, CN, CO₂R¹¹, or perfluoroalkyl having 1 to about 20 carbon atoms; n is an integer of 0 to 12; q is an integer of 0 to 12 when R¹⁰ is H, CO₂R¹¹ or perfluoroalkyl; q is an integer of 1 to 12 wherein R¹⁰ is CN; and R¹¹ is C₁ to C₁₂ alkyl or cycloalkyl, C₆ to C₂₀ aryl, or combinations thereof.

The unsaturated compound can be an acyclic, aliphatic, monoethylenically unsaturated compound or cyclic monoethylenically unsaturated compound, or combinations of two or more thereof. Nonlimiting examples of ethylenically unsaturated compounds are shown in Formulas V and VI, and the corresponding terminal nitrile compounds produced are illustrated by Formulas VII and VIII, respectively, wherein numerically designated radicals have the same meaning and where R⁹ is the same as disclosed above.

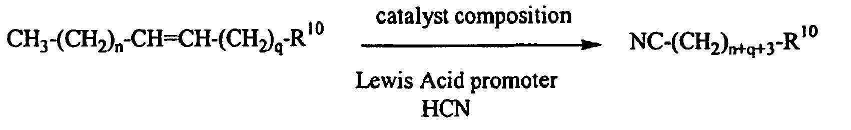

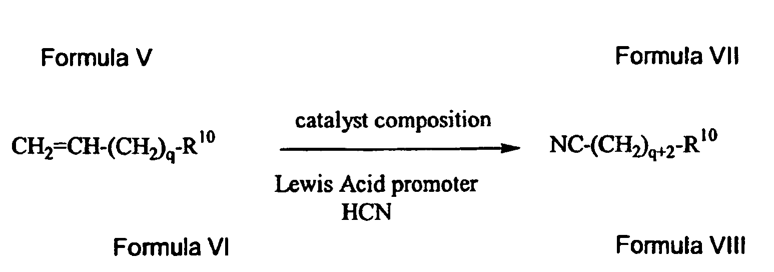

Preferably, the unsaturated organic compounds contain less than 100 ppm peroxides. Examples of suitable ethylenically unsaturated compounds include ethylene, propylene, 1-butene, 2-pentene, 2-hexene, cyclohexene, cyclopentene, allene, 3-pentenenitrile, 4-pentenenitrile, methyl 3-pentenoate, C_{b}F_{2b+1}, where b is an integer of up to 20 and combinations of two or more thereof. The monoethylenically unsaturated compounds can also be conjugated to an ester group such as methyl 2-pentenoate. Preferred olefins are linear alkenes, linear alkenenitriles, linear alkenoates, linear 2-alkenoates, perfluoroalkyl ethylenes, and combinations of two or more thereof. Most preferred substrates include 3-and 4-pentenenitrile, alkyl 2-, 3-, and 4-pentenoates, and C_{b}F_{2b+1}CH=CH₂ (where b is 1 to 12), and combinations of two or more thereof. 3-pentenenitrile and 4-pentenenitrile are especially preferred olefins.

When non-conjugated acyclic aliphatic monoethylenically unsaturated compounds are used, up to about 10% by weight of the monoethylenically unsaturated compound can be present in the form of a conjugated isomer, which itself may undergo hydrocyanation. For example, when 3-pentenenitrile is used, as much as 10% by weight thereof may be 2-pentenenitrile (as used herein, the term "pentenenitrile" is intended to be identical with "cyanobutene"). Suitable unsaturated compounds include unsubstituted hydrocarbons, as well as hydrocarbons substituted with groups that do not attack the catalyst, such as the cyano group.

The process of this invention can be carried out in the presence of one or more Lewis acid promoters that affect both the activity and the selectivity of the catalyst system. The promoter may be an inorganic or organometallic compound in which the cation is selected from scandium, titanium, vanadium, chromium, manganese, iron, cobalt, copper, zinc, boron, aluminum, yttrium, zirconium, niobium, molybdenum, cadmium, rhenium, lanthanum, europium, ytterbium, tantalum, and samarium, and tin. Examples include ZnBr₂, ZnI₂, ZnCl₂, ZnSO₄, CuCl₂, CuCl, Cu(O₃SCF₃)₂, CoCl₂, Col₂, FeI₂, FeCl₃, FeCl₂, FeCl₂(THF)₂, TiCl₄(THF)₂, TiCl₂, ClTi(OiPr)₂, MnCl₂, ScCl₃, AlCl₃, (C₈H₁₇)AlCl₂, (C₈H₁₇)₂AlCl, (iso-C₄H₉)₂AlCl, Ph₂AlCl, PhAlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, (C₆H₅)₃SnX, where X=CF₃SO₃, CH₃C₆H₅SO₃, or (C₆H₅)₃BCN, B(C₆H₅)₃, and TaCl₅. Suitable promoters are further described in U. S. Pat. Nos. 3,496,217; 3,496,218 and 4,774,353, the disclosures of which are incorporated herein. These include metal salts (such as ZnCl₂, CoI₂, and SnCl₂), and organometallic compounds (such as R¹²AlCl₂, R¹²SnO₃SCF₃, and R¹²B, where R¹² is an alkyl or aryl group). U.S. Pat. No. 4,874,884, incorporated herein by reference, describes how synergistic combinations of promoters can be chosen to increase the catalytic activity of the catalyst system. Preferred promoters include CdCl₂, FeCl₂, ZnCl₂, B(C₆H₅)₃, and (C₆H₅)₃Sn(CF₃SO₃), CH₃C₆H₅SO₃, or (C₆H₅)₃BCN. The mole ratio of promoter to Group VIII transition metal present in the reaction can be within the range of about 1:16 to about 50:1.

Hydrocyanation can also be carried out with a conjugated, unsaturated compound. With a conjugated, unsaturated compound, a Lewis Acid promoter is optional. Examples of conjugated, unsaturated compounds containing from about 4 to about 15, preferably 4 to 10 carbon atoms are 1,3 -butadiene, *cis* and *trans*-2,4-hexadienes, *cis* and *trans*-1,3-pentadienes, 1,3-cyclooctadiene and combinations of two or more thereof. Butadiene is especially preferred by reason of its commercial importance in the production of adiponitrile. Preferably, the butadiene contains less than 5 ppm *t*-butyl catechol, less than 500 ppm vinylcyclohexene, and less than 100 ppm peroxides.

The following Formulas IX and X illustrate some suitable starting conjugated olefins. 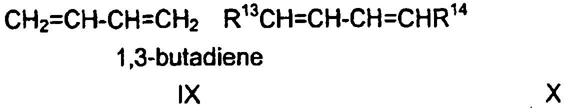 wherein each of R¹³ and R¹⁴, independently, is H or a C₁ to C₃ alkyl. Formulas XI, XII, and XIII represent the products obtained from 1,3-butadiene and HCN: 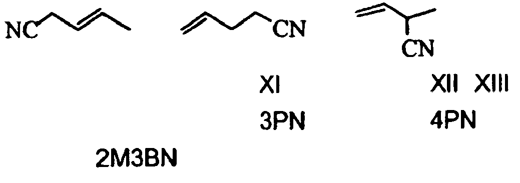 where 3PN is 3-pentenenitrile, 4PN is 4-pentenenitrile, and 2M3BN is 2-methyl-3-butenenitrile.

The reaction of a conjugated unsaturated compound and a HCN-containing fluid can be carried out in the same manner as that described above in relation to monoethylenically unsaturated compounds.

### Isomerization Using Polymeric, Phosphorus-Containing Ligands

In the ninth aspect of the present invention, the polymeric ligand compositions of the present invention may be used to form catalysts, which may be used for the isomerization of branched nitriles to linear nitriles. The isomerization comprises contacting an alkenyl nitrile with a catalyst disclosed above under conditions sufficient to isomerize the alkenyl nitrile. The process can be run with or without a Lewis acid. Examples of suitable alkenyl nitriles include, but are not limited to, 2-alkyl-3-monoalkenenitriles, 3-atkenenitriles, or combinations thereof. The alkenyl nitrile may be produced by a batch or continuous hydrocyanation process. The isomerization can be carried out under substantially similar conditions as described above in relation to hydrocyanation. Preferably, the branched nitriles contain less than 100 ppm peroxides.

A 2-alkyl-3-monoalkenenitrile used as the starting material in the isomerization can be made by the hydrocyanation of a diolefin as described above or can come from any other available sources. The olefinic double bond in the 2-alkyl-3-monoalkenenitriles used as starting materials in the isomerization cannot be conjugated to the triple bond of the cyano group. Suitable starting 2-alkyl-3-monoalkenenitriles can also carry groups that do not attack the catalyst, including, for example, another cyano group. Preferably, the starting 2-alkyl-3-monoalkenenitriles contain from 5 to 8 carbon atoms, excluding any additional substitution. 2-Methyl-3-butenenitrile is an especially important starting material, because it is used to produce adiponitrile. Other representative nitrile starting materials include 2-ethyl-3-butenenitrile and 2-propyl-3-butenenitrile.

When the starting nitrile is 2-methyl-3-butenenitrile (2M3BN, Formula XIII), the isomerization products are those shown in Formulas XI and XII, above.

The isomerization process of this invention can be carried out, for example, at atmospheric pressure and at any temperature in the range of 10 to 200°C, preferably in the range of 60 to150°C. The pressure is not critical, however, and can be above or below atmospheric pressure, if desired. Any of the conventional batch or continuous flow procedures may be used either in the liquid phase or in the vapor phase (with respect to the relatively volatile 2-methyl-3-butenenitrile reactant and linear pentenenitrile products). The reactor may be of any mechanically and chemically resistant material, and is usually of glass or an inert metal or alloy, such as nickel, copper, silver, gold, platinum, stainless steel, Monel® metal alloy or Hastelloy® metal alloy.

The process can be carried out in the absence or in the presence of a solvent or diluent. Any solvent or diluent that is inert to, or nondestructive of, the catalyst can be used. Suitable solvents include, but are not limited to, aliphatic or aromatic hydrocarbons (hexane, cyclohexane, benzene), ethers (diethyl ether, tetrahydrofuran, dioxane, glycol dimethyl ether, anisole), esters (ethyl acetate, methyl benzoate, nitriles (acetonitrile, benzonitrile), or combinations of two or more thereof.

The catalyst (complex of Group VIII metal, preferably nickel, and polymeric ligand) is essentially nonvolatile, whereas the 2-methyl-3-butenenitrile reactant and the linear pentenenitrile products are relatively volatile. Accordingly, in a continuous flow procedure, the catalyst can be a component of the flowing system in a slurry-liquid-phase operation. It can also be in a mobile non-flowing liquid state in a semi-vapor phase operation, or it may be in a fixed-bed state in a conventional flowing vapor-phase operation or flowing liquid-phase operation.

The time required for the isomerization process to obtain a practical level of conversion of, for example, 2-alkyl-3-monoalkenenitrile, to linear alkenenitrile is dependent upon the temperature of reaction, i.e., operation at lower temperature generally requires a longer time than operation at a higher temperature. A practical reaction time can be in the range of a few seconds to many hours (2 seconds to about 24 hours), depending on the particular conditions and method of operation.

The molar ratio of 2-alkyl-3-monoalkenenitrile to catalyst is generally greater than 1:1, usually in the range from about 5:1 to 20,000:1, preferably 100:1 to 5,000:1, for a batch or continuous operation.

### Hydroformylation using Polymeric, Phosphorus-Containing Ligands

In the tenth aspect of the present invention, the polymeric ligands of the present invention may be used to form catalysts which may be used for hydroformylation of monoethylenically unsaturated organic compounds with 2 to 20 carbon atoms to produce corresponding aldehydes. The catalyst comprises a Group VIII metal or Group VIII metal compound combined with at least one polymeric ligand of the present invention. Preferred Group VIII metals for hydroformylation reactions are rhodium, iridium, and platinum, the most preferred being rhodium. The Group VIII metal may be in the form of a compound, such as a hydride, halide, organic acid salt, ketonate, inorganic acid salt, oxide, carbonyl compound, amine compound, or combinations of two or more thereof. Preferred Group VIII metal compounds are Ir₄(CO)₁₂, IrSO₄, RhCl₃, Rh(NO₃)₃, Rh(OAc)₃, Rh₂O₃, Rh(acac)(CO)₂, [Rh(OAc)(COD)]₂, Rh₄(CO)₁₂, Rh₆(CO)₁₆, RhH(CO)(Ph₃P)₃, [Rh(OAc)(CO)₂]₂, [RhCl(COD)]₂, and combinations of two or more thereof ("acac" is an acetylacetonate group; "OAc" is an acetyl group; "COD" is 1,5-cyclooctadiene; and "Ph" is a phenyl group). However, it should be noted that the Group VIII metal compounds are not necessarily limited to the above listed compounds. Rhodium compounds suitable for hydroformylation can be prepared or generated according to techniques well known in the art, as described, for example, in PCT Pat. App. WO 9530680, U. S. Pat. No. 3,907,847, and J. Am. Chem. Soc., 1993, 115, 2066, incorporated herein by reference. Rhodium compounds that contain ligands that can be displaced by the present polymeric phosphite ligands are a preferred source of rhodium. Examples of such preferred rhodium compounds are Rh(CO)₂ (acac), Rh(CO)₂(C₄H₉COCHCO-*t*-C₄H₉), Rh₂O₃, Rh₄(CO)₁₂, Rh₆(CO)₁₆, Rh(O₂CCH₃)₂, Rh(2-ethylhexanoate), and combinations of two or more thereof.

The amount of transition metal in the catalyst may be varied and may be determined by balancing catalyst activity and process economy. In general, the molar ratio of polymeric ligand to transition metal generally can be from about 1:1 to about 100:1, preferably from about 2:1 to about 20:1, moles phosphorus per mole metal.

The reactant of the hydroformylation process is an unsaturated organic compound having at least one "C=C" bond in the molecule and preferably 2 to about 20 carbon atoms. Examples of suitable ethylenically unsaturated organic compounds include, but are not limited to, linear terminal olefinic hydrocarbons (i.e., ethylene, propylene, 1-butene, 1-pentene, 1-hexene, 1-octene, 1-nonene, 1-decene, 1-tetradecene, 1-hexadecene, 1-octadecene, 1-eicosene and 1-dodecene), branched terminal olefinic hydrocarbons (i.e., isobutene and 2-methyl-1-butene); linear internal olefinic hydrocarbons (i.e., *cis-* and *trans*-2-butene, *cis*- and *trans* -2-hexene, *cis*- and *trans* -2-octene, and *cis*- and *trans* -3-octene); branched internal olefinic hydrocarbons (2,3-dimethyl-2-butene, 2-methyl-2-butene and 2-methyl-2-pentene); terminal olefinic hydrocarbons; internal olefinic hydrocarbon mixtures (i.e., octenes, prepared by dimerization of butenes); cyclic olefins (i.e., cyclohexene, and cyclooctene); and combinations of two or more thereof. Preferably, the unsaturated organic compound contains less than 100 ppm peroxides.

Examples of suitable olefinic compounds also include those substituted with an unsaturated hydrocarbon group, including olefinic compounds containing an aromatic substituent such as styrene, alphamethylstyrene and allylbenzene.

The unsaturated organic compound may also be substituted with one or more functional groups containing a heteroatom, such as oxygen, sulfur, nitrogen or phosphorus. Examples of these heteroatom-substituted, ethylenically unsaturated organic compounds include vinyl methyl ether, methyl oleate, oleyl alcohol, 3-pentenenitrile, 4-pentenenitrile, 3-pentenoic acid, 4-pentenoic acid, methyl 3-pentenoate, 7-octen-1-al, acrylonitrile, acrylic acid esters, methyl acrylate, methacrylic acid esters, methyl methacrylate, acrolein, allyl alcohol, 3-pentenal, 4-pentenal, and combinations of two or more thereof.

The hydroformylation process of the invention can be illustrated as follows:

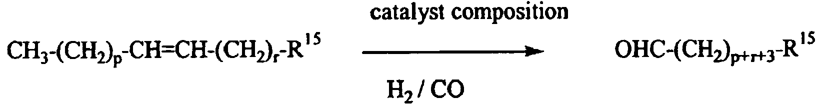

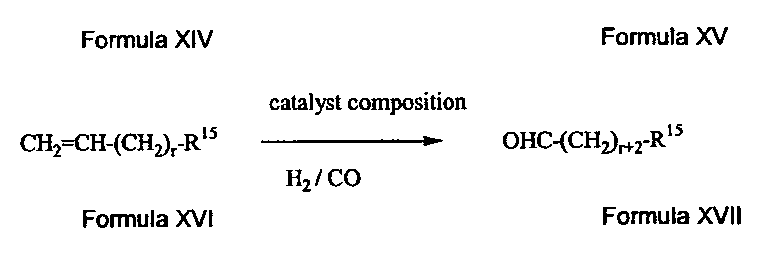

In the above equations, R¹⁵ is H, -CN, -CO₂R¹⁶, -C(O)N(R¹⁶)₂, - CHO, -OR¹⁶, OH, or combinations of two or more thereof; p is an integer from 0 to 12; and r is an integer from 0 to 12. Each R¹⁶ is independently selected from the group consisting of H, C₁ to C₂₀ branched or straight chain alkyl, C₁ to C₂₀ cycloalkyl, and C₆ to C₂₀ aryl.

Particularly preferred unsaturated organic compounds are 3-pentenenitrile, 3-pentenoic acid, 3-pentenal, allyl alcohol, and alkyl 3-pentenoate, such as methyl 3-pentenoate, and combinations of two or more thereof. Preferably, the 3-pentenenitrile, 3-pentenoic acid, 3-pentenal, allyl alcohol, and alkyl 3-pentenoate contain less than 100 ppm peroxides. The linear aldehyde compound prepared by the present process starting with one of these compounds can be used advantageously in the preparation of ε-caprolactam, hexamethylenediamine, 6-aminocaproic acid, 6-aminocapronitrile or adipic acid, which are precursors for nylon-6 and/or nylon-6,6.

The hydroformylation process of the invention also can be carried out with a mixture that comprises two or more unsaturated organic compounds. For example, 3-pentenenitrile can be present in a mixture containing 4-pentenenitrile. Because the 4- isomer reacts in a similar fashion as the corresponding 3-isomer to the desired linear aldehyde, a mixture of isomers can be used directly in the present process.

The 3-pentenenitrile may be present in mixtures containing impurities that do not interfere with the hydroformylation reaction. An example of such an impurity is 2-pentenenitrile.

The hydroformylation process of the invention can be carried out by any means known to one skilled in the art, such as, for example, the one disclosed in U. S. Pat. No. 4,769,498, incorporated herein by reference. Generally, the process can be carried out under any condition sufficient to effect the production of a desired aldehyde. For example, the temperature can be from about 0°C to 200°C, preferably from about 50 to 150°C, and more preferably from 85° to 110°C. The pressure may vary from atmospheric pressure to 5 MPa, preferably from 0.1 to 2 MPa. The pressure is, as a rule, equal to the combined hydrogen and carbon monoxide partial pressures. Inert gases also may be present; the pressure may vary from atmospheric pressure to 15 MPa when inert gases are present. The molar ratio of hydrogen to carbon monoxide is generally between 10:1 and 1:10, and preferably between 6:1 and 1:2 moles hydrogen/mole carbon monoxide. It is most preferred that a 1:1 ratio of carbon monoxide to hydrogen is used.

The amount of catalyst is selected so that favorable results can be obtained with respect to catalyst activity and process economy. In general, the amount of transition metal in the reaction, which comprises an unsaturated organic compound, a catalyst composition, and solvent (if present), can be between 10 and 10,000 ppm and more preferably between 50 and 1,000 ppm, calculated as free metal.

The solvent may be the mixture of reactants of the hydroformylation reaction itself, such as the starting unsaturated compound, the aldehyde product and/or by-products. Other suitable solvents include saturated hydrocarbons (for example, kerosene, mineral oil, or cyclohexane), ethers (for example, diphenyl ether or tetrahydrofuran), ketones (for example, acetone, cyclohexanone), nitriles (for example, acetonitrile, adiponitrile or benzonitrile), aromatics (for example, toluene, benzene, or xylene), esters (for example, methyl valerate, caprolactone), dimethylformamide, or combinations of two or more thereof.

The hydroformylation process can be run in solution or in the gas phase. When the hydroformylation is carried out in the vapor phase, the preferred temperature range is from about 50°C to about 180°C, most preferably from about 90°C to 110°C. The temperature must be chosen high enough to maintain all of the reactants and products in the vapor phase, but low enough to prevent deterioration of the catalyst. The particular preferred temperature depends to some extent on the catalyst being used, the olefinic compound being used, and the desired reaction rate. The operating pressure is not particularly critical and can be from about 0.1 to 1.0 MPa. The pressure and temperature combination must be chosen so as to maintain reactants and products in the vapor phase. A given catalyst is loaded into a reactor, such as a tubular reactor, taking care to avoid exposure of oxygen-sensitive catalysts to oxygen from the air. A gaseous mixture of the desired olefinic compound, carbon monoxide and hydrogen, along with any desired diluent, such as nitrogen, helium or argon, is then passed through the reactor while contacting the catalyst. The reaction products are generally liquid at room temperature and are conveniently recovered by cooling. The reactor effluent can be directly connected to a sampling valve and can be analyzed by gas chromatography. Aldehydic products, such as linear and branched butyraldehydes obtained from hydroformylation of propylene, can be quantitatively separated and analyzed using a 30M DB-Wax® capillary GC column.

For the hydrocyanation, isomerization, and hydroformylation processes described herein, a non-oxidizing environment is desirable in order to retard oxidative deactivation of the catalyst. Accordingly, an inert atmosphere, e.g., nitrogen, is preferably used, although air can be used, if desired, at the expense of loss of a proportion of the catalyst activity through oxidation. Impurities that are detrimental to the catalyst should be kept to a minimum.

The following examples are presented to further illustrate specific features and embodiments of the present invention including various methods of preparing the polymeric substrate on which the phosphorus-containing ligand is to be formed and methods of forming the phosphorus-containing ligand compositions. Similarly the specific reactions and compounds when identfied structurally by chemical formula are intended to be illustrative of the reaction pathway for the formation of phosphorus-containing bidentate ligand compositions of interest. As such, it should be appreciated that other species and distribution of products will be present as generally known in the art and that any performance data associated with such compositions was derived using the mixture as produced without isolation or separation of the specific compound. All parts, proportions, and percentages are by weight, unless otherwise indicated.

In evaluating the performance of the respective phosphorus-containing ligand compositions, the following general procedures were employed unless otherwise noted:

*Preparation of catalyst:* A catalyst solution is prepared by adding 0.0039 grams of Ni(COD)₂ (0.014 mmol) dissolved in 0.320 mL toluene to a specified quantity of the respective phosphorus-containing polymeric ligand composition being evaluated dissolved in 0.200 mL THF.

*Hydrocyanation of butadiene:* A specified volume of the above catalyst solution nominally containing about 0.0020 mmol Ni is added to each of 2 reaction vials fitted with septum caps. The vials are cooled to - 20°C and 120 µL of a solution of HCN in valeronitrile (0.830 mmol HCN) and 280 µL of a solution of butadiene in toluene (0.925 mmol BD) are added to each vial. The vials are sealed and placed in a hot block reactor set at 80°C. Samples are removed after 1.5 and 3 hours and quenched by cooling to -20°C. The reaction mixtures are then diluted in ethyl ether and the product distribution analyzed by GC against valeronitrile as an internal standard. The results are presented as relative percent of the starting HCN that had been converted to useful nitriles (3-pentenenitrile (3PN), and 2-methyl-3-butenenitrile (2M3BN)).

*Isomerization of 2-methyl-3-butene nitrile (2M3BN):* A specified volume of the above catalyst solution containing nominally about 0.002 mmol Ni is added to 2 reaction vials fitted with septum caps. 130 µL of a cold solution containing 2M3BN and valeronitrile (0.930 mmol 2M3BN) are added to each vial. The vials are sealed and placed in a hot block reactor set at 125°C. Samples are removed after 1.5 and 3 hrs, cooled and diluted in ethyl ether. GC using valeronitrile as an internal standard is employed to analyze the product distribution. The results are presented as the 3PN/2M3BN ratio.

*Hydrocyanation of 3-pentenenitrile (3PN):* A specified volume of the above catalyst solution nominally containing about 0.003 mmol Ni and 13 µL of a solution of ZnCl₂ in 3PN (0.0067 mmol ZnCl₂) are added to a vial fitted with a septum cap. The vial is cooled to -20°C and 125 µL of a solution of HCN, 3PN, and 2-ethoxyethyl ether (0.396 mmol HCN, 0.99 mmol 3PN) are added. The 3PN used for hydrocyanation and hydroformylation container approximately 97% *t*-3-pentenenitrile (GC). The vial is sealed and set aside for 24 hours at room temperature. The reaction mixture is diluted with ethyl ether and the product distribution is analyzed by GC using 2-ethoxyethyl ether as an internal standard. The results are presented as the relative percent of the starting pentenenitriles that have been converted to dinitrile product and the percent yield based on HCN. The selectivity to the linear adiponitrile (ADN) isomer is reported as percent ADN in reaction product mixture.

In some examples of hydrocyanation of 3-pentenenitrile, the HCN was added batchwise at the beginning of the experiment and the mixture was heated in a hot block reactor. In some examples, the HCN was added slowly over the course of the experiment and the mixtures were heated in a thermostatically controlled oil bath. HCN was delivered to the flask as an HCN/N₂ gas mixture by bubbling dry nitrogen gas through liquid HCN at 0°C (maintained in a 0°C circulating bath), providing a vapor stream which is about 35% HCN (vol/vol). The rate of nitrogen gas flow determines the rate of HCN delivery. Samples were periodically analyzed by gas chromatography (GC).

### EXAMPLES

### Example 1

### Aspect 5: Polymer derived from the oxidative coupling of 4,4'-ethylidenebis(2-isopropyl-5-methyl)phenol:

To a 25 mL vial with a magnetic stirrer was added 1.0 g of 4,4'-ethylidenebis(2-isopropyl-5-methyl)phenol (prepared as described in Bull. Chem. Soc. Jpn., 1989, 62, 3603) and 50 mg of Cu(OH)Cl(TMEDA),
where TMEDA is tetramethylethylenediamine, prepared according to Tetrahedron Lett., 1994, 35, 7983, and 3 mL of methylene chloride. The mixture was stirred overnight, an additional 6 mL of methylene chloride was added, and the mixture was stirred for two more days. The mixture was treated with an aqueous solution of sodium EDTA (ethylenediaminetetraacetic acid). The aqueous layer was removed and the solid filtered and washed with acetone. After drying under vacuum, 0.833 g of a brown solid was obtained.

### Example 1A

### Aspect 1: Preparation of polymeric phosphite ligand from the reaction of polymer with the phosphorochloridite of o-cresol:

A 100 mL round bottom flask with a magnetic stir bar was charged with 490 mg of the brown solid polymer described in Example 1 and 841 mg of the phosphorochlorodite of o-cresol in about 30 mL of tetrahydrofuran (THF). About 1 g of tri-*n*-butylamine was added. The mixture was stirred overnight and the solvent removed under vacuum. Acetonitrile was added and the solid was filtered, washed with acetonitrile and vacuum dried to give 713 mg of a yellow solid.

### Example 1B

### Preparation and use of catalyst with polymer from Example 1A:

*Aspect 7: Preparation of catalyst:* 0.034 g of the polymeric phosphite ligand described in Example 1 A was weighed into a reaction vial equipped with a septum cap. 200 µL of THF were added to the vial and the sample was shaken. A catalyst solution was prepared by adding 0.0039 g of Ni(COD)₂ (0.014 mmol) in 0.320 mL toluene to the vial.
*Aspect 8: Hydrocyanation of butadiene:* A catalyst sample prepared as above was cooled to -20°C and 280 L of a solution of butadiene in toluene (0.925 mmol butadiene) and 120 L of a solution of HCN in valeronitrile (0.830 mmol HCN) were added. The mixture was heated at 80°C. After 3 hours, a sample was removed and quenched by cooling to -20°C. The mixture was then diluted in ethyl ether and the product distribution analyzed by GC against valeronitrile as internal standard. Analysis showed that 64% of the starting HCN had been converted to useful nitriles (the 3-pentenenitrile/2-methyl-3-butenenitrile, 3PN/2M3BN, ratio was 16.3).
*Aspect 8: Hydrocyanation of 3-pentenenitrile (3PN):* Another catalyst sample prepared as above was cooled to -20°C and 125 µL of a solution of HCN, 3PN, and 2-ethoxyethyl ether (0.396 mmol HCN, 0.99 mmol 3PN) were added. 13 µL of a solution of ZnCl₂ in 3PN (0.0067 mmol ZnCl₂) were added to the vial. The vial was sealed and set aside for 24 hours at room temperature. The reaction mixture was diluted with ethylether and the product distribution analyzed by GC using 2-ethoxyethyl ether as an internal standard. Analysis showed that 66% of the starting HCN had been converted to dinitrile product. The selectivity to the linear ADN isomer was 97.6%.
*Aspect 9: Isomerization of 2-methyl-3-butenenitrile (2M3BN):* To another catalyst sample prepared as above was added 130 L of a cold solution containing 2M3BN (0.930 mmol) and valeronitrile. The mixture was heated to 120°C for 3 hours. GC analysis with valeronitrile as an internal standard indicated that a 3PN/2M3BN ratio of 19.2 had been reached.

### Example 2

### Aspect 1: Preparation of polymer resulting from the coupling of 2,2'-bis(methoxy)-1,1'-binaphthyl-3,3'-diboronic acid and 4,4'-dibromobiphenyl ether:

Under nitrogen atmosphere, an aqueous solution of K₂CO₃ (10 mL, 1 M) was added to a mixture of 2,2'-bis(methoxy)-1,1'-binapthyl-3,3'-diboronic acid (0.880 g, 2.2 mmol), 4,4'-dibromobiphenyl ether (0.722 g, 2.2 mmol), Pd(PPh₃)₄ (0.050 g, 0.044 mmol) and 1,4-dioxane (15 mL), where PPh₃ is triphenyl phosphine. The resulting mixture was refluxed for 48 hours. The organic layer was separated and then diluted with CH₂Cl₂ (125 mL). The solution was washed with 1N HCl (50 mL) and a saturated NaCl solution (2 x 50 mL) and dried over MgSO₄. The solvent was removed by rotary evaporation to give a yellow/tan solid. The solid was redissolved in CH₂Cl₂ and precipitated twice with methanol. The isolated tan solid was dissolved in CH₂Cl₂ and cooled to -30°C. With vigorous stirring, BBr₃ (1.00 mL, 10.6 mmol) was added dropwise. The color of the reaction mixture changed to orange/red after addition of 2-3 drops of BBr₃. During the addition, the temperature was controlled to ensure reaction temperature remained less than -10°C. The reaction mixture was stirred at room temperature for 18 hours and then was cooled to 0°C. Cold distilled water (150 mL) was added dropwise to the reaction mixture and was stirred for 45 minutes resulting in a yellow mixture. The organic layer was separated and washed with 1 N HCl (2 x 125 mL), saturated NaCl solution (150 mL), and dried over MgSO₄. Upon removal of solvent, an orange/brown crystalline solid (0.737 g, 1.2 mmol, 53.8% yield) was isolated. Gel Permeation Chromatography (GPC) in THF with polystyrene standards: M_{w}= 2172 and Mₙ= 1081 (PDI= 2.0), where M_{w} is weight average molecular weight, Mₙ is number average molecular weight and PDI is polydispersity index, calculated as the ratio M_{w}/Mₙ.

### Example 2A:

### Aspect 1: Preparation of polymeric phosphite ligand by reaction of polymer from Example 2 with the phosphorochloridite of 1,2,3,4-tetrahydro-1-naphthol:

Under nitrogen atmosphere, a round bottom flask containing the phosphorochloridite of 1,2,3,4-tetrahydro-1-naphthol (0.76 g, 2.10 mmol) and diethyl ether (10 mL) was cooled to -30°C. The polymeric binaphthol diol as described in Example 2 (0.525 g, 4.58 mmol) was added to the above cold solution followed by the addition of triethylamine (0.3 mL, 2.10 mmol). The resulting mixture was allowed to warm to room temperature and stirred overnight. The solid was filtered, washed with acetonitrile and dried under vacuum to yield a white solid (1.03 g, 96% yield). ³¹P {¹H} NMR (202.4 MHz, C₆D₆): 132.15, 132.81(major). GPC (THF, polystyrene standards): Mₙ = 1,305, M_{w} = 2,272, PDI = 1.74. Elemental Analysis: 68.70 %C, 6.15 %H, 3.18 %P.

### Example 2B

### Preparation and use of catalyst with polymer from Example 2A:

*Aspect 7: Preparation of catalyst:* A catalyst solution was prepared by adding 0.0039 g of Ni(COD)₂ (0.014 mmol) in 0.320 ml toluene to 0.026 g of the ligand described in Example 2A (0.020 mmol) in 0.200 mL THF.
*Aspect 8: Hydrocyanation of butadiene:* 74 µl of the above catalyst solution (0.0020 mmol Ni) were added to each of 2 reaction vials fitted with septum caps. The vials were cooled to -20°C and 120 µl of a solution of HCN in valeronitrile (0.830 mmol HCN) and 280 µl of a solution of butadiene (BD) in toluene (0.925 mmol BD) were added to each vial. The vials were sealed and placed in a hot-block reactor set at 80°C. Samples were removed after 1.5 and 3 hours and quenched by cooling to -20°C. The reaction mixtures were then diluted in ethyl ether and the product distribution analyzed by GC against valeronitrile as an internal standard. Analysis showed that 76.1 and 78.8% of the starting HCN had been converted to useful nitriles (the 3PN/2M3BN ratio was 0.94 after 1.5 hours and also 0.94 after 3 hours).
*Aspect 8: Hydrocyanation of 3-pentenenitrile (3PN):* 116 µl of the above catalyst solution (0.0031 mmol Ni), and 13 µl of a solution of ZnCl₂ in 3PN (0.0067 mmol ZnCl₂) were added to a vial fitted with a septum cap. The vial was cooled to -20°C and 125 µl of a solution of HCN, 3PN, and 2-ethoxyethyl ether (0.396 mmol HCN, 0.99 mmol 3PN) were added. The vial was sealed and set aside for 24 hours at room temperature. The reaction mixture was diluted with ethyl ether and the product distribution analyzed by GC using 2-ethoxyethyl ether as an internal standard. Analysis showed that 7.6% of the starting pentenenitriles had been converted to dinitrile product (21% yield based on HCN.) The selectivity to the linear adiponitrile (ADN) isomer was 96.0%.
*Aspect 9: Isomerization of 2-methyl-3-butene nitrile (2M3BN):* 82 µl of the above catalyst solution (0.0022 mmol Ni) were added to each of 2 reaction vials fitted with septum caps. 130 µl of a cold solution containing 2M3BN and valeronitrile (0.930 mmol 2M3BN) were added to each vial. The vials were sealed and placed in a hot block reactor set at 125°C. Samples were removed after 1.5 and 3.0 hrs, cooled and diluted in ethyl ether. The product distribution was analyzed using GC with valeronitrile as an internal standard. The 3PN/2M3BN ratio was 0.36 after 1.5 hrs and 0.57 after 3 hours.

### Example 3

### Aspect 1: Preparation of poly(1,1'-bi-2-naphthol):

The synthesis of poly(1,1'-bi-2-naphthol) was performed following Ni catalyzed polymerization of 6,6'-dibromo-2,2'-bis(methoxy)-1,1'-binaphthyl, using procedures described in J. Org. Chem., 1996, 61, 5200.

### Example 3A

### Aspect 1: Preparation of polymeric phosphite ligand from reaction of poly(1,1'-bi-2-naphthol) with phosphorochlorodite of thymol:

Under nitrogen atmosphere, a round bottom flask containing the phosphorochloridite of 2-isopropyl-5-methyl-phenol (thymol, 0.146 g, 0.40 mmol) and diethyl ether (10 mL) was cooled to -30°C. The polymeric binaphthol diol described in Example 3 above (0.050 g, 0.17 mmol) was added to the cold solution, followed by the addition of triethylamine (0.06 mL, 0.43 mmol). The resulting mixture was allowed to warm to room temperature and stirred for an additional two hours. The liquid was decanted from the tan precipitate. The solid was washed with acetonitrile (2 x 10 mL), anhydrous diethyl ether (10 mL) and dried under vacuum to yield a tan solid (0.050 g, 31% yield). ³¹P {¹H} NMR (202.4 MHz, C₆D₆): 131.90 (major), 133.04. The polymer was not soluble in THF and was only slightly soluble in benzene.

### Example 3B

### Preparation and use of catalyst with polymer from Example 3A:

*Aspect 7: Preparation of catalyst:* 0.018 g (0.019 mmol) of the polymeric phosphite ligand prepared in Example 3A was weighed into a reaction vial equipped with a septum cap. 200 µL of THF were added to the vial and the sample was shaken. A catalyst solution was prepared by adding 0.0039 g of Ni(COD)₂ (0.014 mmol) in 0.320 mL toluene to the vial.
*Aspect 8: Hydrocyanation of 3-pentenenitrile (3PN):* The catalyst sample prepared above was cooled to -20°C and 125 µL of a solution of HCN, 3PN, and 2-ethoxyethyl ether (0.396 mmol HCN, 0.99 mmol 3PN) were added. 13 µL of a solution of ZnCl₂ in 3PN (0.0067 mmol ZnCl₂) were added to the vial. The vial was sealed and set aside for 24 hours at room temperature. The reaction mixture was diluted with ethylether. The product distribution was analyzed by GC with 2-ethoxyethyl ether as an internal standard. Analysis showed that 5.7% of the starting pentenenitriles had been converted to dinitrile product (15.7% yield based on HCN.) The selectivity to the linear ADN isomer was 86.7%.

### Example 4

### Aspect 1: Preparation of polymer by reaction of polymer from Example 3 with phosphorochloridite prepared from the acetal of 2,2-dimethyl-1,3-propanediol and salicyaldehyde:

Under nitrogen atmosphere, a round bottom flask containing the phosphorochloridite of the acetal from the reaction of 2,2-dimethyl-1,3-propanediol and salicyaldehyde (0.220 g, 0.45 mmol), diethyl ether (3 mL) and triethylamine (Et₃N) (0.06 mL, 0.43 mmol) was cooled to -30°C. The polymeric binaphthol diol described in Example 3 (0.060 g, 0.20 mmol) was dissolved in diethyl ether (2 mL) and cooled to -30°C. This solution was added to the above phosphorochloridite cold solution. The resulting mixture was allowed to warm to room temperature and stirred for an additional two hours. The liquid was decanted from the tan precipitate. The solid was washed with anhydrous diethyl ether (10 mL) and dried under vacuum to yield a tan solid (0.061 g, 26% yield).

### Example 4A

### Hydrocyanation and isomerization with polymer from Example 4:

*Aspect 7: Preparation of catalyst:* A catalyst solution was prepared by adding 0.0039 g of Ni(COD)₂ (0.014 mmol) in 0.320 ml toluene to 0.084 g of the ligand from Example 4 (0.070 mmol) in 0.200 mL toluene.
*Aspect 8: Hydrocyanation of butadiene:* 74 µl of the above catalyst solution (0.0020 mmol Ni) were added to each of 2 reaction vials fitted with septum caps. The vials were cooled to -20°C and 120 µl of a solution of HCN in valeronitrile (0.830 mmol HCN) and 280 µl of a solution of butadiene (BD) in toluene (0.925 mmol BD) were added to each vial. The vials were sealed and placed in a hot block reactor set at 80°C. Samples were removed after 1.5 and 3 hours and quenched by cooling to -20°C. The reaction mixtures were then diluted in ethyl ether and the product distribution analyzed by GC against valeronitrile as an internal standard. Analysis showed that 33.4 and 43.4% of the starting HCN had been converted to useful nitriles and the 3PN/2M3BN ratio was 0.47 and 0.46, after 1.5 and 3 hours respectively.
*Aspect 8: Hydrocyanation of 3-pentenenitrile (3PN):* 116 µl of the above catalyst solution (0.0031 mmol Ni), and 13 µl of a solution of ZnCl₂ in 3PN (0.0067 mmol ZnCl₂) were added to a vial fitted with a septum cap. The vial was cooled to -20°C and 125 µl of a solution of HCN, 3PN, and 2-ethoxyethyl ether (0.396 mmol HCN, 0.99 mmol 3PN) were added. The vial was sealed and set aside for 24 hours at room temperature. The reaction mixture was diluted with ethyl ether and the product distribution analyzed by GC using 2-ethoxyethyl ether as an internal standard. Analysis showed that 6.4% of the starting pentenenitriles had been converted to dinitrile product (17.7% yield based on HCN.) The selectivity to the linear ADN isomer was 92.6%.
*Aspect 9: Isomerization of 2-methyl-3-butene nitrile (2M3BN):* 82 µl of the above catalyst solution (0.0022 mmol Ni) were added to each of 2 reaction vials fitted with septum caps. 130 µl of a cold solution containing 2M3BN and valeronitrile (0.930 mmol 2M3BN) were added to each vial. The vials were sealed and placed in a hot block reactor set at 125°C. Samples were removed after 1.5 and 3.0 hrs, cooled and diluted in ethyl ether. The product distribution was analyzed by GC using valeronitrile as an internal standard. The 3PN/2M3BN ratio was 0.04 after 1.5 hrs and 0.03 after 3 hours.

### Example 5

### Aspect 1: Process for preparing 3,3'-di-isopropyl-6,6'-dimethyl-2,2'-biphenol:

### Preparation of 4-t-butylthymol:

1 g of concentrated sulfuric acid was added to 30 g (0.20 mol) of thymol, heated at 60°C under nitrogen. After heating to 90°C, a slow stream of isobutylene was introduced over about 2 hours. The reaction stalled at about 50% conversion, so an additional charge of sulfuric acid was added. The reaction was monitored by GC until approximately 70-80% conversion was achieved. The reaction was diluted with water and neutralized with NaHCO₃, and some remaining thymol was removed by distillation. The residue was dissolved in hot hexanes, separated from the aqueous phase, and cooled in an ice-bath. The crude residue was recrystallized from hexanes to afford 20 g of 4-*t*-butylthymol: mp 76-77 °C. ¹H-NMR (CDCl₃) 1.25 (d, 6H, J = 7 Hz), 1.38, (s, 9H), 2.44 (s, 3H), 3.15 (septet, 1H), 4.49 (s, 1H), 6.51 (s, 1H), 7.18 (s, 1H).

### Preparation of 5,5'-di-t-butyl-3,3'-di-isopropyl-6,6'-dimethyl-2,2'-biphenol :

To a solution of 20 g (0.104 mol) of 4-*t*-butylthymol in 50 mL of dichloromethane was added 1.0 g (5 mmol) of copper chlorohydroxide-TMEDA complex. The dark purple mixture was allowed to stir under ambient air for three days. The mixture was diluted with hexanes, washed with aqueous EDTA solution, dried over MgSO₄ and concentrated to dryness. The residue was chromatographed on silica gel to give 3.6 g of pure dimer, 5,5'-di-*t*-butyl-3,3'-di-isopropyl-6,6'-dimethyl-2,2'-biphenol, mp 105-108 °C, ¹H-NMR (CDCl₃) 1.26 (d, 6H),1.43, (s, 9H), 3.25 (septet, 1H), 4.58 (s, 1H), 7.30 (s, 1H).

### Example 5A

### Aspect 1: Reaction of 3,3'-di-isopropyl-6,6'-dimethyl-2,2'-biphenol with acetaldehyde:

To a 100 mL round bottom flask was added 0.248 g of acetaldehyde, 1.68 g of 3,3'-di-isopropyl-6,6'-dimethyl-2,2'-biphenol and 15 mL of nitromethane. The mixture was cooled in an ice bath and 1 mL of concentrated HCl in 5 mL of nitromethane was added dropwise. After 10 minutes, the ice bath was removed and 10 mL more of nitromethane was added. The mixture was stirred for two days and then refluxed for 6 hours. The solid was filtered and washed with toluene. The entire solid dissolved in toluene. The solvent was removed to give 1.849 g of a brown solid. Gel permeation chromatography (GPC) in THF with polystyrene standards indicated Mₙ of 1326 and M_{w} of 1518 (PDI = 1.14).

### Example 5B

### Aspect 1: Preparation of polymeric ligand from the reaction of polymer from Example 5A with the phosphorochlorodite of o-cresol:

A vial was charged with 300 mg of the polymer from Example 5A, 543 mg of phosphorochlorodite of o-cresol and 15 mL of toluene. The mixture was cooled to -30°C and 350 mg of triethylamine was added. The mixture was stirred overnight at room temperature, filtered through silica gel and the solvent removed to give 776 mg of a brown oil.

### Example 5C

### Preparation and use of catalyst with polymer from Example 5B:

*Aspect 7: Preparation* of *catalyst:* A catalyst solution was prepared by adding 0.0039 g of Ni(COD)₂ (0.014 mmol) in 0.320 ml toluene to 0.033 g of the above ligand (0.042 mmol) in 0.200 mL toluene.
*Aspect 8: Hydrocyanation of butadiene:* 74 µl of the above catalyst solution (0.0020 mmol Ni) were added to each of 2 reaction vials fitted with septum caps. The vials were cooled to -20°C and 120 µl of a solution of HCN in valeronitrile (0.830 mmol HCN) and 280 µl of a solution of butadiene (BD) in toluene (0.925 mmol BD) were added to each vial. The vials were sealed and placed in a hot block reactor set at 80°C. Samples were removed after 1.5 and 3 hours and quenched by cooling to -20°C. The reaction mixtures were diluted in ethyl ether and the product distribution analyzed by GC against valeronitrile as an internal standard. Analysis showed that 36 and 62% of the starting HCN had been converted to useful nitriles, with 3PN/2M3BN ratios of 4.38 and 16.0, after 1.5 and 3 hours respectively.
*Aspect 8: Hydrocyanation of 3-pentenenitrile (3PN):* 116 µl of the above catalyst solution (0.0031 mmol Ni), and 13 µl of a solution of ZnCl₂ in 3PN (0.0067 mmol ZnCl₂) were added to a vial fitted with a septum cap. The vial was cooled to -20°C and 125 µl of a solution of HCN, 3PN, and 2-ethoxyethyl ether (0.396 mmol HCN, 0.99 mmol 3PN) were added. The vial was sealed and set aside for 24 hours at room temperature. The reaction mixture was diluted with ethyl ether and the product distribution analyzed by GC using 2-ethoxyethyl ether as an internal standard. Analysis showed that 77.6% of the starting HCN had been converted to dinitrile. The selectivity to the linear ADN isomer was 97.3%.
*Aspect 9: Isomerization of 2-methyl-3-butene nitrile (2M3BN):* 82 µl of the above solution (0.0022 mmol Ni) were added to each of 2 reaction vials fitted with septum caps. 130 µl of a cold solution containing 2M3BN and valeronitrile (0.930 mmol 2M3BN) were added to each vial. The vials were sealed and placed in a hot block reactor set at 125°C. Samples were removed after 1.5 and 3.0 hrs, cooled and diluted in ethyl ether. The product distribution was analyzed using GC with valeronitrile as an internal standard. The 3PN/2M3BN ratio was 16.6 after 1.5 hrs and 16.7 after 3 hours.

### Example 6

### Aspect 1: Reaction of 3,3',4,4',6,6'-hexamethyl-2,2'-biphenol with 4,4'-bis(chloromethyl)-1,1'-biphenyl; reaction of the product with the phosphorochlorodite of o-cresol:

A mixture containing 558 mg of 4,4'-bis(chloromethyl)-1,1'-biphenyl, 600 mg of 3,3',4,4',6,6'-hexamethyl-2,2'-biphenol, and 4 mL of methylene chloride was heated at 50°C overnight. The starting 3,3',4,4',6,6'-hexamethyl-2,2'-biphenol was prepared by the coupling of 2,3,5-trimethylphenol using the literature procedure for the preparation of 3,3', 5,5'-tetramethyl-2,2'-biphenol (J. Org. Chem., 1963, 28, 1063).

A purple gel formed; 12 mL of methylene chloride and 20 mL of water were added. The aqueous layer was decanted and 25 mL of acetone was added to give a beige solid. The mixture was stirred for 30 minutes and filtered, washed with acetone and vacuum dried to give 0.819 g of brown solid. In a dry box, a 50 mL flask with a magnetic stir bar was charged with 508 mg of the brown solid from above and 20 mL of THF. The mixture was stirred for 2 hours at room temperature. To this mixture was added 656 mg of the phosphorochloridite of *o*-cresol. The mixture was cooled to -30°C and 700 mg of tributylamine was slowly added. The mixture was stirred overnight. Acetonitrile (10 mL) was added and the solvent was removed by vacuum. Another 10 mL of acetonitrile was added and the slurry was stirred for two hours. The off-white solid was filtered and vacuum dried to give 1.02 g of off-white solid. This solid was mixed with 8 mL of acetonitrile for two days, filtered, and vacuum dried to give 865 mg of off-white solid.

### Example 6A

*Aspect 7: Preparation of catalyst:* To 39 mg of the off-white solid obtained as in Example 6 was added 320 L of a solution containing 39 mg of Ni(COD)₂ in 2.79 g toluene.
*Aspect 8: Hydrocyanation of butadiene:* The catalyst mixture was cooled to -20°C and 280 L of a solution of BD in toluene (0.925 mmol BD) and 120 L of a solution of HCN in valeronitrile (0.830 mmol HCN) were added. The mixture was heated at 80°C. After 3 hours, a sample was removed and quenched by cooling to -20°C. The mixture was then diluted in ethyl ether and the product distribution analyzed by GC against valeronitrile as internal standard. Analysis showed that 37% of the starting HCN had been converted to useful nitriles (3PN/2M3BN = 4.4).
*Aspect 8: Hydrocyanation of 3-pentenenitrile:* Another catalyst sample was prepared as above, and 125 L of a solution of HCN, 3PN and 2-ethoxyethyl ether (0.396 mmol HCN, 0.99 mmol 3PN) was added. To this mixture was added 13 L of a solution of ZnCl₂ in 3PN (0.0067 mmol ZnCl₂). The mixture was set aside for 24 hours at room temperature. GC analysis using 2-ethoxyethyl ether as an internal standard showed 27.2% of the starting pentenitriles had been converted to dinitrile product (68% yield based on HCN). The selectivity to the linear ADN isomer was 97.6%.
*Aspect 9: Isomerization of 2-methyl-3-butenenitrile:* Another catalyst sample was prepared as above, and 130 L of a cold solution containing 2M3BN (0.930 mmol) and valeronitrile was added. The mixture was heated to 120°C for 3 hours. GC analysis with valeronitrile as an internal standard indicated 3PN/2M3BN ratio of 18.7.

### Example 6B

*Aspect 8: Hydrocyanation of 3-pentenenitrile:* A flask was charged with 259 mg of the polymer from Example 6, 5 mL of THF and 0.058 g of Ni(COD)2. The mixture was stirred for 30 minutes and the solvent was removed under vacuum. To the residue was added 0.029 g of zinc chloride, 5 mL of 3PN, and 5 mL of toluene. The mixture was treated with HCN with a nitrogen flow rate of 12 cc/min at 50°C; after 180 minutes, GC analysis indicated 86.9% ADN, 3.5% MGN and 0.3% ESN.

### Example 7

### Aspect 1: Polymeric phosphite from the reaction of binaphthol and 4,4'-bis(chloromethyl)phenyl:

A vial with a magnetic stir bar was charged with 2 g binaphthol, 1.754 g of 4,4'-bis(chloromethyl)-1,1'-phenyl, 100 mg of zinc chloride and 10 mL of methylene chloride. The mixture was heated at 50°C for 3 days. The solid was collected and washed with 25 mL of water and then with acetone. After drying, 2.627 g of a brown solid was obtained. To a flask was added 1.360 g of the brown solid and 20 mL of THF. After stirring overnight, an additional 5 mL of THF was added along with 1.636 g of the phosphorochloridite of o-cresol and 1.665 g of tri-n-butylamine in 15 mL of THF. The mixture was stirred overnight and the solvent was removed under vacuum. Acetonitrile was added and the solid filtered and washed with acetonitrile to give 2.34 g of light brown solid. Elemental Analysis: 54.93 %C; 2.40 %H; 0.21 %CI; 6.42 %P. To 1.500 g of this light brown solid was added 20 mL of THF and 0.288g of Ni(COD)₂. The mixture was stirred overnight and the solvent was removed under vacuum.

### Example 7A

### Hydrocyanation and isomerization results with nickel catalyst from Example 7:

*Aspect 8: Hydrocyanation of butadiene:* A reaction vial was charged with 9 mg of the catalyst described above and cooled to -20°C. 120 µL of a solution of HCN in valeronitrile (0.830 mmol HCN) and 280 µL of a solution of BD in toluene (0.925 mmol BD) were added to the vial. The vial was sealed and placed in a hot block reactor set at 80°C. The reaction mixture was quenched after 3 hours by cooling to -20°C. The reaction mixture was diluted in ethyl ether and the product distribution analyzed by GC against valeronitrile as an internal standard. GC analysis indicated that 87% of the starting HCN had been converted to useful nitriles, with 3PN/2M3BN of 10.83.
*Aspect 8: Hydrocyanation of 3-pentenenitrile:* 359 mg of the nickel catalyst from Example 7, 0.029 g of zinc chloride, 5 mL of toluene, and 5 mL of 3PN were treated with HCN at 24 cc/min of nitrogen at 50°C for 90 min. GC analysis indicated 68.9% ADN, 10.7% MGN and 1.7% ESN. Upon standing under nitrogen overnight, GC analysis indicated 73.7% ADN; 11.7% MGN and 1.9% of ESN.
*Aspect 9: Isomerization of 2-methyl-3-butene nitrile (2M3BN):* 130 µL of a cold solution containing 2M3BN and valeronitrile (0.930 mmol 2M3BN) were added to 9 mg of the catalyst above in a reaction vial. The vial was sealed and placed in a hot block reactor set at 125°C. After 3.0 hrs, the reaction mixture was cooled and diluted in ethyl ether. GC using valeronitrile as an internal standard indicated a 3PN/2M3BN ratio of 12.7.

### Example 8

### Aspect 1: Preparation of polymer from reaction of poly(1,1'-bi-2-naphthol) with phosphorochlorodite of 4-chloro-1-naphthol:

Under nitrogen atmosphere, a round bottom flask containing the phosphorochloridite of 4-chloro-1-naphthol (1.93 g, 4.58 mmol) and diethyl ether (12 mL) was cooled to -30°C. The polymeric binaphthol diol described in Example 3 (0.525 g, 1.83 mmol) was added to the above cold solution, followed by the addition of triethylamine (0.6 mL, 4.60 mmol). The resulting mixture was allowed to warm to room temperature and stirred overnight. The liquid was decanted from the white precipitate. The precipitate was washed with acetonitrile (3 x 5 mL), anhydrous diethyl ether (10 mL) and dried under vacuum to yield a white solid (1.58 g, 90% yield). ³¹P {¹H} NMR (202.4 MHz, C₆D₆): 126.58, 131.05 (major). ³¹P {¹H} NMR (202.4 MHz, C₆D₆): 132.15, 132.81 (major). Gel Permeation Chromatography (GPC) in THF with polystyrene standards: Mₙ = 6,089, M_{w} = 14,213, PDI = 2.33. Elemental Analysis: 65.87 %C, 5.01 %H, 3.16 %P.

### Example 8A

### Aspect 10: Hydroformylation of 3PN with polymeric phosphite from Example 8:

A 100 mL autoclave was charged with 0.600 g of polymeric phosphite as described in Example 8. The autoclave was evacuated and a solution containing 0.038 g of Rh(CO)₂(acac), 2 g of 1,2-dichlorobenzene and 70 g of 3PN was loaded under vacuum. The autoclave was pressurized with 0.45 MPa CO/H₂ (1:1 molar ratio), heated at 95°C under vigorous stirring for 6 hours while flowing CO/H₂ at a rate of 20 ml/min for 6 hours. Samples were removed from the reactor and analyzed by gas chromatography on an HP 5890A Chromatograph with a DB5 fused silica capillary column (30 meters, 0.32 mm I.D., 0.25 um film thickness) purchased from J.B. Scientific.

| Time (min) | Conversion (%) | Linearity (%) | Selectivity (%) |
|---|---|---|---|
| 15 | 1.56 | 62.43 | 45.5 |
| 30 | 3.06 | 65.38 | 52.29 |
| 60 | 4.13 | 64.99 | 45.60 |
| 90 | 6.05 | 65.92 | 47.54 |
| 120 | 8.28 | 66.87 | 49.09 |
| 175 | 12.01 | 67.67 | 50.20 |
| 240 | 20.43 | 68.93 | 51.96 |

### Example 9

### Aspect 1: Preparation of polymeric phosphite from reaction of poly(1,1'-bi-2-naphthol) with phosphorochlorodite of 2-phenyl-phenol:

Under nitrogen atmosphere, a round bottom flask containing the phosphorochloridite of 2-phenylphenol which contained 30% of P[O-(2-Ph)Ph]₃ (0.415 g, 1.02 mmol) and diethyl ether (10 mL) was cooled to -30 °C. The polymeric binaphthol diol described in Example 3 (0.100 g, 0.35 mmol) was added to the above cold solution and followed by the addition of triethylamine (0.12 mL, 0.87 mmol). The resulting mixture was allowed to warm to room temperature and stirred overnight. The liquid was decanted from the white solid. This solid was washed with acetonitrile (3 x 5 mL), anhydrous diethyl ether (10 mL) and dried under vacuum to yield a white solid (0.162 g, 45 % yield). ³¹P {¹H} NMR (202.4 MHz, C₆D₆): 131.11 (major), 154.19. This compound was partially soluble in benzene and acetonitrile.

### Example 9A

### Aspect 10: Hydroformylation of 3PN with polymeric phosphite from Example 9:

In a drybox, a solution containing 3PN (5.0 g), Rh(CO)₂(acac) (2.5 mg), and 1,2-dichlorobenzene (internal standard, 0.27 M) was prepared. This solution was added to a glass-lined pressure vessel containing approximately two equivalents of polymeric phosphite, as described in Example 9, per equivalent of rhodium. The reactor was sealed, pressurized to 65 psi with a 1:1 molar ratio of CO/H₂ and heated to 95°C for 3 hours. The reactor was cooled and depressurized. A sample of the reaction mixture was analyzed by gas chromatography on a HP 5890A Chromatograph with a DB5 fused silica capillary column (30 meters, 0.32 mm I.D., 0.25 um film thickness) purchased from J.B. Scientific. GC analysis: 73.9 % conversion; selectivity to 5-formylvaleronitrile: 59.9 % on a mole basis; linearity of aldehydes produced: 68.0 %.

### Example 10

### Aspect 1: Preparation of polymeric phosphite by reaction of poly(1,1'-bi-2-naphthol) with phosphorochlorodite of 2-tetrahydropyran-2-yl-phenol:

Under nitrogen atmosphere, a round bottom flask containing the phosphorochloridite of 2-tetrahydropyran-2-yl-phenol (0.338 g, 0.80 mmol) and diethyl ether (10 mL) was cooled to -30°C. The polymeric binaphthol diol described in Example 3 (0.100 g, 0.35 mmol) was added to the above cold solution and followed by the addition of triethylamine (0.12 mL, 0.87 mmol). The resulting mixture was allowed to warm to room temperature and stirred overnight. The liquid was decanted from the white solid. This solid was washed with acetonitrile (3 x 5 mL), anhydrous diethyl ether (10 mL) and dried under vacuum to yield a white solid (0.139 g, 38 %) yield. This compound is insoluble in benzene.

### Example 10A

### Aspect 10: Hydroformylation of 3PN with polymeric phosphite from Example 10:

In a drybox, a solution was prepared containing 3PN (5.0 g), Rh(CO)₂(acac) (2.5 mg), and 1,2-dichlorobenzene (internal standard, 0.27 M). This solution was added to a glass-lined pressure vessel containing approximately two equivalents of polymeric phosphite, as described in Example 10, per equivalent of rhodium. The reactor was sealed, pressurized to 65 psi with a 1:1 molar ratio of CO/H₂ and heated to 95°C for 3 hours. The reactor was cooled and depressurized. A sample of the reaction mixture was analyzed by gas chromatography on a HP 5890A Chromatograph with a DB5 fused silica capillary column (30 meters, 0.32 mm I.D., 0.25 um film thickness) purchased from J.B. Scientific. GC analysis: 69.1 % conversion; selectivity to 5-formylvaleronitrile: 56.3 % on a mole basis; linearity of aldehydes produced: 68.0 %.

### Example 11

### Aspect 1: Copolymerization of vinylbenzyl chloride and 3,3'-diisopropyl-6,6'-dimethyl-2,2'biphenol:

3,3'-Diisopropyl-6,6'-dimethylbiphenol (0.5 g), ZnCl₂ (0.1 g) and 1,2-dichloroethane (3 ml) were placed in a vial and stirred for 10 minutes at room temperature. Vinylbenzyl chloride (2.0 g) was added to the vial. The vial was closed and heated in an oil bath at 60°C for 90 minutes. The vial was opened at 10, 30, and 60 minutes after initiation of heating to release any pressure build-up. The resulting brown solid was washed with MeOH (2 x 20 ml), EtOAc (2 x 20 ml), and dried under vacuum to give a solid, which was treated with AlCl₃ in toluene (4 ml) at 60°C for 2 hours. The polymer was collected by filtration, washed with hexanes (2 x 20 ml), and MeOH (2 x 20 ml), and vacuum dried to provide 1.96 g of polymer.

### Example 11A

### Aspect 1: Preparation of polymeric, phosphorus-containing composition from the polymer of Example 11 and phosphorochloridite of o-cresol:

A mixture of 0.62 g of the polymer from Example 11 and the phosphorochloridite of o-cresol (1.0 g) was mixed in 20 ml THF. Tributylamine (1.0 g) was added and the mixture was stirred for 3 days at room temperature. The solid was collected by filtration, washed with acetonitrile (3 x 20 ml), and vacuum dried to give 0.72 g of the ligand. Elemental Analysis: 1.8 %P.

### Example 11B

*Aspect 7: Preparation of catalyst:* To 74 mg of the off-white solid obtained in Example 11A was added 320 L of a solution containing 39 mg of Ni(COD)₂ in 2.79 g toluene.
*Aspect 8: Hydrocyanation of butadiene:* The catalyst mixture was cooled to -20°C and 280 L of a solution of butadiene in toluene (0.925 mmol butadiene) and 120 L of a solution of HCN in valeronitrile (0.830 mmol HCN) were added. The mixture was heated to 80°C. After 3 hours, a sample was removed and quenched by cooling to -20°C. The mixture was then diluted in ethyl ether and the product distribution analyzed by GC against valeronitrile as internal standard. Analysis showed that 89% of the starting HCN had been converted to useful nitriles (3PN/2M3BN = 1.1).
*Aspect 9: Isomerization of 2-methyl-3-butenenitrile:* A catalyst sample was prepared as above. 130 L of a cold solution containing 2M3BN (0.930 mmol) and valeronitrile were added. The mixture was heated to 120°C for 3 hours. GC analysis with valeronitrile as an internal standard indicated 3PN/2M3BN ratio of 0.32.

### Example 12

### Aspect 3: Preparation of phosphite ligand by reaction of 6,6'-dibromo-1,1'-bi-2-naphthol with the phosphorochloridite of 1,2,3,4-tetrahydro-1-naphthol:

Under nitrogen atmosphere, a round bottom flask containing the phosphorochloridite of 1,2,3,4-tetrahydro-1-naphthol (0.76 g, 2.10 mmol) and diethyl ether (10 mL) will be cooled to -30°C. The 6,6'-dibromo-1,1'-bi-2-naphthol (0.47 g, 1.1 mmol) will be added to the above cold solution followed by the addition of triethylamine (0.3 mL, 2.10 mmol). The resulting mixture will be allowed to warm to room temperature and stirred for a couple of hours. The white precipitate will be filtered through celite/alumina and the resulting clear solution will be concentrated to yield the desired phosphite ligand.

### Example 12A

### Preparation of polymer resulting from the coupling of phosphite ligand described in Example 12 and 1,4-phenyldiboronic acid:

Under a nitrogen atmosphere, a mixture comprising phosphite ligand from Example 12 (2.4g g, 2.2 mmol), 1,4-phenyldiboronic acid (0.36 g, 2.2 mmol), Pd(PPh₃)₄ (0.050 g, 0.044 mmol), K₂CO₃ (0.83 g, 6 mmol) and toluene (15 mL), where PPh₃ is triphenyl phosphine will be placed in a flask equipped with a reflux condenser. The resulting mixture will be refluxed for 48 hours. The solution will be filtered through celite and then the solvent will be removed by rotary evaporation to give the phosphite polymer.

Having thus described and exemplified the invention with a certain degree of particularity, it should be appreciated that the following claims are not to be so limited but are to be afforded a scope commensurate with the wording of each element of the claim and equivalents thereof.

### Preferred Embodiments

According to further preferred embodiments, there is provided:
1. A process for preparing a polymeric, phosphorus-containing composition by:
   (1) preparing a polymeric precursor by polymerizing at least one compound of Formula I and/or at least one compound of Formula II, 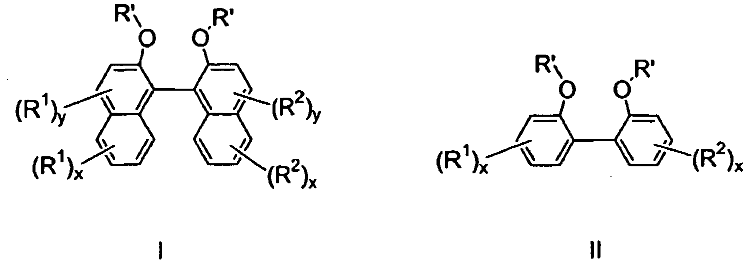 wherein:
      x = 0 to 4;
      y = 0 to 2;
      and each R' is individually hydrogen or a hydroxyl protective group selected from alkyl, alkoxyalkyl, carbonylalkyl, or a crown ether formed by taking both R' groups together;
      each R¹ and R² are individually hydrogen, linear or branched alkyl, cycloalkyl, acetal, ketal, aryl, alkoxy, cycloalkoxy, aryloxy, ester, nitrile, fluorine, chlorine, bromine, perhaloalkyl, hydrocarbylsulfinyl, hydrocarbylsulfonyl, formyl, hydrocarbylcarbonyl and cyclic ether;
      provided at least two R¹ or at least two R² or at least one R¹ and at least one R² are capable of reacting with one another to cause aryl to aryl coupling of at least one substituted 2,2'-dihydroxyl-1,1'-binaphthalene as shown in Formula I and/or the substituted 2,2'-dihydroxyl-1,1'-biphenylene as shown in Formula II;
      and wherein said aryl to aryl coupling is achieved by:
      i. polymerization to form a carbon to carbon bond linking at least one compound of Formula I and/or at least one compound of Formula II; or
      ii. copolymerization of at least one compound of Formula I and/or at least one compound of Formula II in the presence of an aryl comonomer having at least at least one R¹ and at least one R² capable of reacting with at least one compound of Formula I and/or at least one compound of Formula II; or
      iii. copolymerization of at least one compound of Formula I and/or at least one compound of Formula II by a Friedel-Crafts reaction of at least one compound of Formula I and/or at least one compound of Formula II in the presence of at least one dialkylating or dibenzylating or diacylating comonomer; or
      iv. copolymerization of at least one compound of Formula II with an aldehyde; and,
   (2) if R' is a hydroxyl protective group, converting R' to H, or alkali metal, or alkaline metal and,
   (3) phosphonylating the product of step (1) if R' is other than a hydroxyl protective group, or the product of steps (1) and (2) if R' is a hydroxyl protective group, with at least one diaryloxyphosphite [-P(-OAr)₂], diarylphosphine [-P(Ar)₂], and/or aryl,aryloxyphosphinite [-P(Ar)(-OAr)];
   where each Ar is individually phenyl or naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide;
   and each Ar can be further substituted with C₁ to C₂₀ branched or straight chain alkyl, C₁ to C₂₀ cycloalkyl, C₆ to C₂₀ aryl, acetal, ketal, cycloalkoxy, aryloxy, perhaloalkyl, fluorine, chlorine, bromine, formyl, ester, hydrocarbylsulfinyl, hydrocarbylsulfonyl, hydrocarbylcarbonyl, cyclic ether, -OR³, -CO₂R³, -SO₃R³, -S(O)R³, -SO₂R³, -CHO, -C(O)R³, or CN;
   where each R³ is independently C₁ to C₂₀ branched or straight chain alkyl, and C₁ to C₂₀ cycloalkyl, or C₆ to C₂₀ aryl.
2. A polymeric, phosphorus-containing composition made as described in embodiment 1.
3. A process for making a polymeric, phosphorus-containing composition by copolymerization of a composition comprising at least one substituted phosphonylated 2,2'-dihydroxyl-1,1'-binaphthalene as shown in Formula III and/or at least one substituted phosphonylated 2,2'-dihydroxyl-1,1'-biphenylene as shown in Formula IV with an aryl comonomer containing at least two boronic acid functional groups, a dihydroxy aryl bridging group and/or a diamine aryl bridging group to effect aryl to aryl coupling and produce the phosphite-containing polymer, 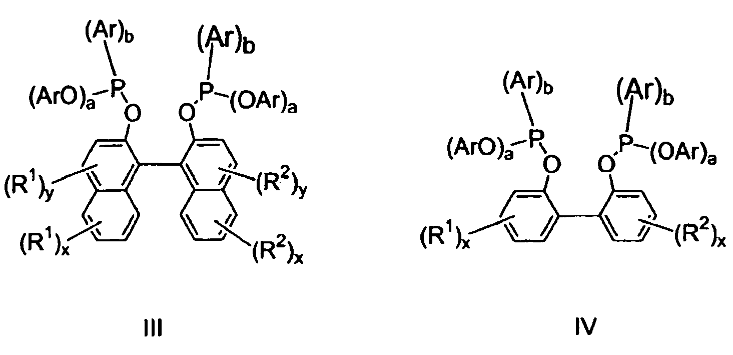 wherein:
   x = 0 to 4;
   y = 0 to 2;
   a and b are individually either 0, 1, or 2, provided a+b = 2;
   each Ar is individually phenyl or naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide;
   each Ar can be further substituted with C₁ to C₂₀ branched or straight chain alkyl, C₁ to C₂₀ cycloalkyl, C₆ to C₂₀ aryl, acetal, ketal, cycloalkoxy, aryloxy, perhaloalkyl, fluorine, chlorine, bromine, formyl, ester, hydrocarbylsulfinyl, hydrocarbylsulfonyl, hydrocarbylcarbonyl, cyclic ether, -OR³, -CO₂R³, -SO₃R³, -S(O)R³, -SO₂R³, -CHO, -C(O)R³, and CN; where each R³ is independently C₁ to C₂₀ branched or straight chain alkyl, and C₁ to C₂₀ cycloalkyl, or C₆ to C₂₀ aryl;
   and each R¹ and R² are individually hydrogen, linear or branched alkyl, cycloalkyl, acetal, ketal, aryl, alkoxy, cycloalkoxy, aryloxy, ester, amine, boronic acid, boronic ester, nitrile, fluorine, chlorine, bromine, perhaloalkyl, hydrocarbylsulfinyl, hydrocarbylsulfonyl, formyl, hydrocarbylcarbonyl or cyclic ether,
   provided at least two R¹ or at least two R² or at least one R¹ and at least one R² are capable of reacting with one another to cause aryl to aryl coupling of at least one of the substituted phosphonylated 2,2'-dihydroxyl-1,1'-binaphthalene as shown in Formula III and/or the substituted 2,2'-dihydroxyl-1,1'-biphenylene as shown in Formula IV.
4. A polymeric, phosphorus-containing composition made as described in embodiment 3.
5. A process for preparing a polymeric, phosphorus-containing composition by:
   (1) aryl to aryl oxidative coupling of monomer having the structure: 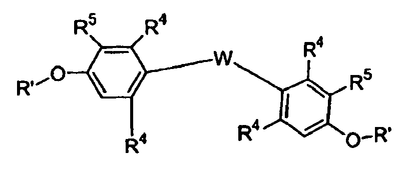 wherein:
      W is selected from C₆-C₂₀ arylene, a C₁-C₂₀ alkylene and cycloalkylene;
      each R' is individually hydrogen or a hydroxyl protective group selected from, but not limited to, alkyl, alkoxyalkyl, carbonylalkyl, or a crown ether formed by taking both R' groups together;
      each R⁴ is independently H, C₁ to C₂₀ branched or straight chain alkyl, C₁ to C₂₀ cycloalkyl, or C₆ to C₂₀ aryl;
      each R⁵ is independently C₁ to C₂₀ branched or straight chain alkyl, C₁ to C₂₀ cycloalkyl, or C₆ to C₂₀ aryl; and
   (2) converting R' with a substituent selected from diaryloxyphosphite [-P(-O-Ar)₂], diarylphosphine [-P(Ar)₂], and/or aryl,aryloxyphosphinite [-P(Ar)(-O-Ar)], where each Ar is individually phenyl, substituted phenyl, naphthyl, or substituted naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide; and each Ar can be further substituted with C₁ to C₂₀ branched or straight chain alkyl, C₁ to C₂₀ cycloalkyl, C₆ to C₂₀ aryl, acetal, ketal, cycloalkoxy, aryloxy, perhaloalkyl, fluorine, chlorine, bromine, formyl, ester, hydrocarbylsulfinyl, hydrocarbylsulfonyl, hydrocarbylcarbonyl, cyclic ether, -OR³, -CO₂R³, -SO₃R³, -S(O)R³, - SO₂R³, -CHO, -C(O)R³, and CN; where each R³ is independently C₁ to C₂₀ branched or straight chain alkyl, and C₁ to C₂₀ cycloalkyl, or C₆ to C₂₀ aryl.
6. A polymeric, phosphorus-containing composition made as described in embodiment 5.
7. A catalyst composition comprising at least one polymeric, phosphorus-containing composition of embodiment 2, embodiment 4 or embodiment 6 and at least one Group VIII metal.
8. The catalyst composition of embodiment 7 wherein the Group VIII metal is nickel, palladium, cobalt.
9. The catalyst composition of embodiment 7 wherein the Group VIII metal is rhodium, iridium and platinum.
10. The catalyst composition of embodiment 8 wherein the Group VIII metal is nickel.
11. The catalyst composition of embodiment 8 or of embodiment 10 further comprising a Lewis acid.
12. The catalyst composition of embodiment 11 wherein the Lewis acid is selected from the group consisting of ZnBr₂, ZnI₂, ZnCl₂, ZnSO₄, CuCl₂, CuCl, Cu(O₃SCF₃)₂, CoCl₂, CoI₂, FeI₂, FeCl₃, FeCl₂, FeCl₂(THF)₂, TiCl₄ (THF)₂, TiCl₂, ClTi(OiPr)₂, MnCl₂, ScCl₃, AlCl₃, (C₈H₁₇)AlCl₂, (C₈H₁₇)₂AlCl, (iso-C₄H₉)₂AlCl, Ph₂AlCl, PhAlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, (C₆H₅)₃SnX, where X=CF₃SO₃, CH₃C₆H₅SO₃, or (C₆H₅)₃BCN, B(C₆H₅)₃, and TaCl₅,
13. The catalyst composition of embodiment 12 wherein the Lewis acid is zinc chloride or iron chloride.
14. A hydrocyanation process comprising contacting an unsaturated organic compound with HCN in the presence of a catalyst composition comprising at least one composition of embodiment 2, embodiment 4 or embodiment 6 and at least one Group VIII metal, and optionally a Lewis acid.
15. The hydrocyanation process of embodiment 14 wherein the Lewis acid is selected from the group consisting of ZnBr₂, ZnI₂, ZnCl₂, ZnSO₄, CuCl₂, CuCl, Cu(O₃SCF₃)₂, CoCl₂, CoI₂, FeI₂, FeCl₃, FeCl₂, FeCl₂(THF)₂, TiCl₄ (THF)₂, TiCl₂, ClTi(OiPr)₂, MnCl₂, ScCl₃, AlCl₃, (C₈H₁₇)AlCl₂, (C₈H₁₇)₂AlCl, (iso-C₄H₉)₂AlCl, Ph₂AlCl, PhAlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, (C₆H₅)₃SnX, where X=CF₃SO₃, CH₃C₆H₅SO₃, or (C₆H₅)₃BCN, B(C₆H₅)₃, and TaCl₅.
16. The hydrocyanation process of embodiment 15 wherein the Lewis acid is zinc chloride or iron chloride.
17. The hydrocyanation process of embodiment 16 wherein the Group VIII metal is nickel, palladium or cobalt.
18. The hydrocyanation process of embodiment 17 wherein the unsaturated organic compound is 3-pentenenitrile or 4-pentenenitrile and the Group VIII metal is nickel.
19. The hydrocyanation process of embodiment 18 wherein the HCN contains less than 20 ppm sulfur dioxide, less than 40 ppm sulfuric acid, less than 20 ppm cyanogen, less than 10 ppm epoxide, less than 20 ppm acrylonitrile, and less than 100 ppm peroxides, and the pentenenitriles contain less than 100 ppm peroxides.
20. The hydrocyanation process of embodiment 19 wherein the Group VIII metal is nickel and the unsaturated organic compound is 1,3-butadiene.
21. The hydrocyanation process of embodiment 20 wherein the HCN contains less than 20 ppm sulfur dioxide, less than 40 ppm sulfuric acid, less than 20 ppm cyanogen, less than 10 ppm epoxide, less than 20 ppm acrylonitrile, and less than 100 ppm peroxides, and the 1,3-butadiene contains less than 5 ppm *t*-butyl catechol, less than 500 ppm vinylcyclohexene, and less than 100 ppm peroxides.
22. A hydroformylation process comprising contacting an unsaturated organic compound with CO and H₂ in the presence of a catalyst composition comprising at least one composition of embodiment 2, embodiment 4 and/or embodiment 6 and at least one Group VIII metal.
23. The hydroformylation process of embodiment 22 wherein the Group VIII metal is rhodium, iridium or platinum.
24. The hydroformylation process of embodiment 23 wherein the unsaturated organic compound is selected from the group consisting of 3-pentenenitrile, 3-pentenoic acid, 3-pentenal, allyl alcohol, and alkyl 3-pentenoate or mixture thereof.
25. The hydroformylation process of embodiment 24 wherein the unsaturated organic compound contain less than 100 ppm peroxides and the Group VIII metal is rhodium.
26. An isomerization process comprising reacting an unsaturated organic nitrile compound in the presence of a catalyst composition comprising at least one composition of embodiment 2, embodiment 4 and/or embodiment 6 and at least one Group VIII metal.
27. The isomerization process of embodiment 26 wherein the Group VIII metal is nickel, palladium or cobalt.
28. The isomerization process of embodiment 27 wherein the unsaturated organic nitrile compound is 2-methyl-3-butenenitrile and the Group VIII metal is nickel.
29. The isomerization process of embodiment 28 wherein the 2-methyl-3-butenenitrile contains less than 100 ppm peroxides.

## Claims

1. A process for preparing a polymeric, phosphorus-containing composition by:
(1) preparing a polymeric precursor by polymerizing at least one compound of Formula I and/or at least one compound of Formula II, 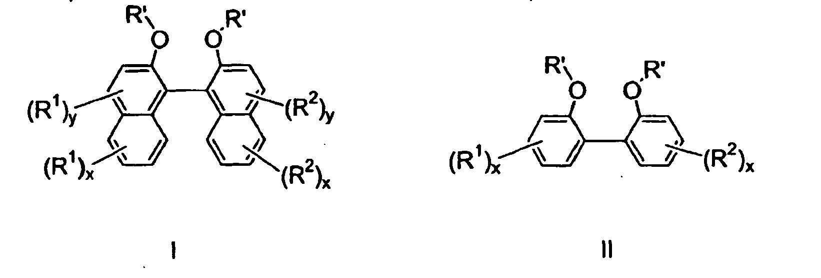 wherein:
x = 0 to 4;
y = 0 to 2:
and each R' is individually hydrogen or a hydroxyl protective group selected from alkyl, alkoxyalkyl, carbonylalkyl, or a crown ether formed by taking both R' groups together;
each R¹ and R² are individually hydrogen, linear or branched alkyl, cycloalkyl, acetal, ketal, aryl, alkoxy, cycloalkoxy, aryloxy, ester, nitrile, fluorine, chlorine, bromine, perhaloalkyl, hydrocarbylsulfinyl, hydrocarbylsulfonyl, formyl, hydrocarbylcarbonyl and cyclic ether;
provided at least two R¹ or at least two R² or at least one R¹ and at least one R² are capable of reacting with one another to cause aryl to aryl coupling of at least one substituted 2,2'-dihydroxyl-1,1'-binaphthalene as shown in Formula I and/or the substituted 2,2'-dihydroxyl-1,1'-biphenylene as shown in Formula II;
and wherein said aryl to aryl coupling is achieved by:
i. polymerization to form a carbon to carbon bond linking at least one compound of Formula I and/or at least one compound of Formula II; or
ii. copolymerization of at least one compound of Formula I and/or at least one compound of Formula II in the presence of an aryl comonomer having at least at least one R¹ and at least one R² capable of reacting with at least one compound of Formula I and/or at least one compound of Formula II; or
iii. copolymerization of at least one compound of Formula I and/or at least one compound of Formula II by a Friedel-Crafts reaction of at least one compound of Formula I and/or at least one compound of Formula II in the presence of at least one dialkylating or dibenzylating or diacylating comonomer; or
iv. copolymerization of at least one compound of Formula II with an aldehyde; and,
(2) if R' is a hydroxyl protective group, converting R' to H, or alkali metal, or alkaline metal and,
(3) phosphonylating the product of step (1) if R' is other than a hydroxyl protective group, or the product of steps (1) and (2) if R' is a hydroxyl protective group, with at least one diaryloxyphosphite [-P(-OAr)₂], diarylphosphine [-P(Ar)₂], and/or aryl,aryloxyphosphinite [-P(Ar)(-OAr)];
where each Ar is individually phenyl or naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide;
and each Ar can be further substituted with C₁ to C₂₀ branched or straight chain alkyl, C₁ to C₂₀ cycloalkyl, C₆ to C₂₀ aryl, acetal, ketal, cycloalkoxy, aryloxy, perhaloalkyl, fluorine, chlorine, bromine, formyl, ester, hydrocarbylsulfinyl, hydrocarbylsulfonyl, hydrocarbylcarbonyl, cyclic ether, -OR³, -CO₂R³, -SO₃R³, -S(O)R³, -SO₂R³, -CHO, -C(O)R³, or CN; where each R³ is independently C₁ to C₂₀ branched or straight chain alkyl, and C₁ to C₂₀ cycloalkyl, or C₆ to C₂₀ aryl.

2. A polymeric, phosphorus-containing composition obtainable according to the process described in Claim 1.

3. A catalyst composition comprising at least one polymeric, phosphorus-containing composition of Claim 2, and at least one Group VIII metal.

4. The catalyst composition of Claim 3 wherein the Group VIII metal is nickel, palladium, cobalt.

5. The catalyst composition of Claim 3 wherein the Group VIII metal is rhodium, iridium and platinum.

6. The catalyst composition of Claim 4 wherein the Group VIII metal is nickel.

7. The catalyst composition of Claim 4 or of Claim 6 further comprising a Lewis acid.

8. The catalyst composition of Claim 1 wherein the Lewis acid is selected from the group consisting of ZnBr₂, Znl₂, ZnCl₂, ZnSO₄, CuCl₂, CuCl, Cu(O₃SCF₃)₂, CoCl₂, CoCl₂, Fel₂, FeCl₃, FeCl₂, FeCl₂(THF)₂, TiCl₄ (THF)_{2,} TiCl₂, ClTi(OiPr)₂, MnCl₂, ScCl₃, AlCl₃, (C₈H₁₇)AlCl₂, (C₈H₁₇)₂AlCl, (iso-C₄H₉)₂AlCl, Ph₂AlCl, PhAlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, (C₆H₅)₃SnX, where X=CF₃SO₃. CH₃C₆H₅SO₃, or (C₆H₅)₃BCN, B(C₆H₅)₃, and TaCl₅.

9. The catalyst composition of Claim 8 wherein the Lewis acid is zinc chloride or iron chloride.

10. A hydrocyanation process comprising contacting an unsaturated organic compound with HCN in the presence of a catalyst composition comprising at least one composition of Claim 2, and at least one Group VIII metal, and optionally a Lewis acid.

11. The hydrocyanation process of Claim 10 wherein the Lewis acid is selected from the group consisting of ZnBr₂, Znl₂. ZnCl₂, ZnSO₄, CuCl₂, CuCl, Cu(O₃SCF₃)₂, CoCl₂, CoCl₂, Fel₂, FeCl₃, FeCl₂, FeCl₂(THF)₂, TiCl₄ (THF)₂, TiCl₂, CiTi(OiPr)₂, MnCl₂, ScCl₃, AlCl_{3,} (C₈H₁₇)AlCl₂, (C₈H₁₇)₂AlCl, (iso-C₄H₉)₂AlCl, Ph₂AlCl, PhAlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, (C₆H₅)₃SnX, where X=CF₃SO₃, CH₃C₆H₅SO₃, or (C₆H₅)₃BCN, B(C₆H₅)₃, and TaCl_{5.}

12. The hydrocyanation process of Claim 11 wherein the Lewis acid is zinc chloride or iron chloride.

13. The hydrocyanation process of Claim 12 wherein the Group VIII metal is nickel, palladium or cobalt.

14. The hydrocyanation process of Claim 13 wherein the unsaturated organic compound is 3-pentenenitrile or 4-pentenenitrile and the Group VIII metal is nickel.

15. The hydrocyanation process of Claim 14 wherein the HCN contains less than 20 ppm sulfur dioxide, less than 40 ppm sulfuric acid, less than 20 ppm cyanogen, less than 10 ppm epoxide, less than 20 ppm acrylonitrile, and less than 100 ppm peroxides, and the pentenenitriles contain less than 100 ppm peroxides.

16. The hydrocyanation process of Claim 15 wherein the Group VIII metal is nickel and the unsaturated organic compound is 1,3-butadiene.

17. The hydrocyanation process of Claim 16 wherein the HCN contains less than 20 ppm sulfur dioxide, less than 40 ppm sulfuric acid, less than 20 ppm cyanogen, less than 10 ppm epoxide, less than 20 ppm acrylonitrile, and less than 100 ppm peroxides, and the 1,3-butadiene contains less than 5 ppm *t*-butyl catechol, less than 500 ppm vinylcyclohexene, and less than 100 ppm peroxides.

18. A hydroformylation process comprising contacting an unsaturated organic compound with CO and H₂ in the presence of a catalyst composition comprising at least one composition of Claim 2, and at least one Group VIII metal.

19. The hydroformylation process of Claim 18 wherein the Group VIII metal is rhodium, iridium or platinum.

20. The hydroformylation process of Claim 19 wherein the unsaturated organic compound is selected from the group consisting of 3-pentenenitrile, 3-pentenoic acid, 3-pentenal, allyl alcohol, and alkyl 3-pentenoate or mixture thereof.

21. The hydroformylation process of Claim 20 wherein the unsaturated organic compound contain less than 100 ppm peroxides and the Group VIII metal is rhodium.

22. An isomerization process comprising reacting an unsaturated organic nitrile compound in the presence of a catalyst composition comprising at least one composition of Claim 2, and at least one Group VIII metal.

23. The isomerization process of Claim 22 wherein the Group VIII metal is nickel, palladium or cobalt.

24. The isomerization process of Claim 23 wherein the unsaturated organic nitrile compound is 2-methyl-3-butenenitrile and the Group VIII metal is nickel.

25. The isomerization process of Claim 24 wherein the 2-methyl-3-butenenitrile contains less than 100 ppm peroxides.

## Patentansprüche

1. Verfahren zur Herstellung einer polymeren, phosphorhaltigen Zusammensetzung durch:
(1) Herstellen eines Polymervorläufers durch Polymerisation mindestens einer Verbindung mit der Formel I und/oder mindestens einer Verbindung mit der Formel II, 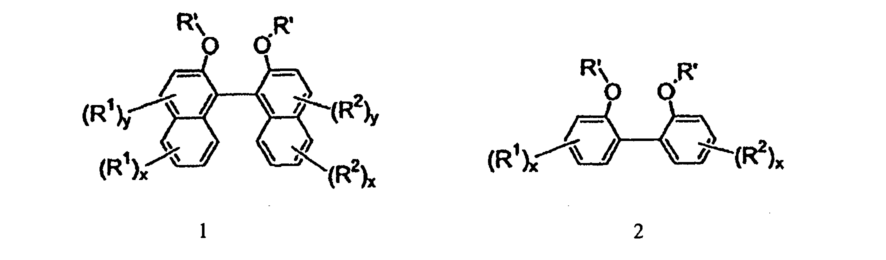 wobei:
x = 0 bis 4 ist;
y = 0 bis 2 ist;
und jedes R' für sich Wasserstoff oder eine Hydroxyl-Schutzgruppe ist, die unter Alkyl, Alkoxyalkyl, Carbonylalkyl oder einem durch Zusammenfassen beider R'-Gruppen gebildeten Kronenether ausgewählt ist;
wobei jedes R¹ und R² für sich Wasserstoff, ein lineares oder verzweigtes Alkyl, Cycloalkyl, Acetal, Ketal, Aryl, Alkoxy, Cycloalkoxy, Aryloxy, Ester, Nitril, Fluor, Chlor, Brom, Perhalogenalkyl, Hydrocarbylsulfinyl, Hydrocarbylsulfonyl, Formyl, Hydrocarbylcarbonyl bzw. ein cyclischer Ether ist;
vorausgesetzt, daß mindestens zwei R¹ oder mindestens zwei R² oder mindestens ein R¹ und mindestens ein R² reaktionsfähig miteinander sind, um eine Aryl-Aryl-Bindung mindestens eines substituierten 2,2'-Dihydroxyl-1,1'-binaphthalins, wie in Formel I dargestellt, und/oder des substituierten 2,2'-Dihydroxyl-1,1'-biphenylens, wie in Formel II dargestellt, zu bewirken;
und wobei die Aryl-Aryl-Bindung erzielt wird durch:
i. Polymerisation zur Bildung einer Kohlenstoff-Kohlenstoff-Bindung, die mindestens eine Verbindung mit der Formel I und/oder mindestens eine Verbindung mit der Formel II bindet; oder
ii. Copolymerisation mindestens einer Verbindung mit der Formel I und/oder mindestens einer Verbindung mit der Formel II in Gegenwart eines Aryl-Comonomers mit mindestens einem R¹ und mindestens einem R², das reaktionsfähig mit mindestens einer Verbindung mit der Formel I und/oder mindestens einer Verbindung mit der Formel II ist; oder
iii. Copolymerisation mindestens einer Verbindung mit der Formel I und/oder mindestens einer Verbindung mit der Formel II durch eine Friedel-Crafts-Reaktion mindestens einer Verbindung mit der Formel I und/oder mindestens einer Verbindung mit der Formel II in Gegenwart mindestens eines dialkylierenden oder dibenzylierenden oder diacylierenden Comonomers; oder
iv. Copolymerisation mindestens einer Verbindung mit der Formel II mit einem Aldehyd; und
(2) wenn R' eine Hydroxyl-Schutzgruppe ist, Umwandlung von R' in H oder ein Alkalimetall oder Erdalkalimetall und
(3) Phosphonylierung des Produkts von Schritt (1), wenn R' keine Hydroxyl-Schutzgruppe ist, oder des Produkts der Schritte (1) und (2), wenn R' eine Hydroxyl-Schutzgruppe ist, mit mindestens einem Diaryloxyphosphit [-P(-O-Ar)₂], Diarylphosphin [-P(Ar)₂] und/oder Aryl, Aryloxyphosphinit [-P(Ar)(-O-Ar)];
wobei jedes Ar für sich Phenyl oder Naphthyl ist, vorausgesetzt, daß die zwei Ar-Gruppen, die direkt oder indirekt an das gleiche Phosphoratom gebunden sind, durch eine unter Direktbindung, Alkyliden, sekundärem oder tertiärem Amin, Sauerstoff, Sulfid, Sulfon und Sulfoxid ausgewählte Bindungseinheit aneinander gebunden werden können;
und wobei jedes Ar ferner mit einem verzweigten oder geradkettigen C₁-C₂₀-Alkyl, C₁-C₂₀-Cycloalkyl, C₆-C₂₀-Aryl, Acetal, Ketal, Cycloalkoxy, Aryloxy, Perhalogenalkyl, Fluor, Chlor, Brom, Formyl, Ester, Hydrocarbylsulfinyl, Hydrocarbylsulfonyl, Hydrocarbylcarbonyl, cyclischen Ether, -OR³, - CO₂R³, -SO₃R³, -S(O)R³, -SO₂R³, -CHO, -C(O)R³ oder CN substituiert werden kann, wobei jedes R³ unabhängig voneinander ein verzweigtes oder geradkettiges C₁-C₂₀-Alkyl und C₁-C₂₀-Cycloalkyl oder C₆-C₂₀-Aryl ist.

2. Polymere phosphorhaltige Zusammensetzung, erhältlich gemäß dem in Anspruch 1 beschriebenen Verfahren.

3. Katalysatorzusammensetzung, umfassend mindestens eine polymere, phosphorhaltige Zusammensetzung nach Anspruch 2 und mindestens ein Metall der Gruppe VIII.

4. Katalysatorzusammensetzung nach Anspruch 3, wobei das Metall der Gruppe VIII Nickel, Palladium, Cobalt ist.

5. Katalysatorzusammensetzung nach Anspruch 3, wobei das Metall der Gruppe VIII Rhodium, Iridium und Platin ist.

6. Katalysatorzusammensetzung nach Anspruch 4, wobei das Metall der Gruppe VIII Nickel ist.

7. Katalysatorzusammensetzung nach Anspruch 4 oder Anspruch 6, weiterhin umfassend eine Lewis-Säure.

8. Katalysatorzusammensetzung nach Anspruch 7, wobei die Lewis-Säure aus der Gruppe ausgewählt ist, bestehend aus ZnBr₂, Znl₂, ZnCl₂, ZnSO₄, CuCl₂, CuCl, Cu(O₃SCF₃)₂, CoCl₂, CoI₂, FeI₂, FeCl₃, FeCl₂, FeCl₂(THF)₂, TiCl₄(THF)₂, TiCl₂, ClTi(OiPr)₂, MnCl₂, ScCl₃, AlCl₂, (C₈H₁₇)AlCl₂, (C₈H₁₇)₂AlCl, (iSO-C₄H₉)₂AlCl, Ph₂AlCl, PhAlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, (C₆H₅)₃SnX, wo X = CF₃SO₃, CH₃C₆H₅SO₃, oder (C₆H₅)₃BCN, B(C₆H₅)₃, und TaCl₅.

9. Katalysatorzusammensetzung nach Anspruch 8, wobei die Lewis-Säure Zinkchlorid oder Eisenchlorid ist.

10. Hydrocyanierungsverfahren, umfassend Inkontaktbringen einer ungesättigten organischen Verbindung mit HCN in Anwesenheit einer Katalysatorzusammensetzung, umfassend mindestens eine Zusammensetzung nach Anspruch 2 und mindestens ein Metall der Gruppe VIII und gegebenenfalls eine Lewis-Säure.

11. Hydrocyanierungsverfahren nach Anspruch 10, wobei die Lewis-Säure aus der Gruppe ausgewählt ist, bestehend aus ZnBr₂, ZnI₂, ZnCl₂, ZnSO₄, CuCl₂, CuCl, Cu(O₃SCF₃)₂, CoCl₂, CoI₂, FeI₂, FeCl₃, FeCl₂, FeCl₂(THF)₂, TiCl₄(THF)₂, TiCl₂, ClTi(OiPr)₂, MnCl₂, ScCl₃, AlCl₃, (C₈H₁₇)AlCl₂, (C₈H₁₇)₂AlCl, (iso-C₄H₉)₂AlCl, Ph₂AlCl, PhAlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, (C₆H₅)₃SnX, wo X = CF₃SO₃, CH₃C₆H₅SO₃, oder (C₆H₅)₃BCN, B(C₆H₅)₃, und TaCl₅.

12. Hydrocyanierungsverfahren nach Anspruch 11, wobei die Lewis-Säure Zinkchlorid oder Eisenchlorid ist.

13. Hydrocyanierungsverfahren nach Anspruch 12, wobei das Metall der Gruppe VIII Nickel, Palladium oder Cobalt ist.

14. Hydrocyanierungsverfahren nach Anspruch 13, wobei die ungesättigte organische Verbindung 3-Pentennitril oder 4-Pentennitril ist und das Metall der Gruppe VIII Nickel ist.

15. Hydrocyanierungsverfahren nach Anspruch 14, wobei das HCN weniger als 20 ppm Schwefeldioxid, weniger als 40 ppm Schwefelsäure, weniger als 20 ppm Cyanogen, weniger als 10 ppm Epoxid, weniger als 20 ppm Acrylnitril und weniger als 100 ppm Peroxide enthält und die Pentennitrile weniger als 100 ppm Peroxide enthalten.

16. Hydrocyanierungsverfahren nach Anspruch 15, wobei das Metall der Gruppe VIII Nickel ist und die ungesättigte organische Verbindung 1,3-Butadien ist.

17. Hydrocyanierungsverfahren nach Anspruch 16, wobei das HCN weniger als 20 ppm Schwefeldioxid, weniger als 40 ppm Schwefelsäure, weniger als 20 ppm Cyanogen, weniger als 10 ppm Epoxid, weniger als 20 ppm Acrylnitril und weniger als 100 ppm Peroxide enthält und das 1,3-Butadien weniger als 5 ppm t-Butylcatechol, weniger als 500 ppm Vinylcyclohexen und weniger als 100 ppm Peroxide enthält.

18. Hydroformylierungsverfahren, umfassend Inkontaktbringen einer ungesättigten organischen Verbindung mit CO und H₂ in Anwesenheit einer Katalysatorzusammensetzung, umfassend mindestens eine Zusammensetzung nach Anspruch 2 und mindestens ein Metall der Gruppe VIII.

19. Hydroformylierungsverfahren nach Anspruch 18, wobei das Metall der Gruppe VIII Rhodium, Iridium oder Platin ist.

20. Hydroformylierungsverfahren nach Anspruch 19, wobei die ungesättigte organische Verbindung aus der Gruppe ausgewählt ist, bestehend aus 3-Pentennitril, 3-Pentensäure, 3-Pentenal, Allylalkohol und Alkyl-3-pentenoat oder einem Gemisch davon.

21. Hydroformylierungsverfahren nach Anspruch 20, wobei die ungesättigte organische Verbindung weniger als 100 ppm Peroxide enthält und das Metall der Gruppe VIII Rhodium ist.

22. Isomerisierungsverfahren, umfassend Umsetzen einer ungesättigten organischen Nitrilverbindung in Anwesenheit einer Katalysatorzusammensetzung, umfassend mindestens eine Zusammensetzung nach Anspruch 2 und mindestens ein Metall der Gruppe VIII.

23. Isomerisierungsverfahren nach Anspruch 22, wobei das Metall der Gruppe VIII Nickel, Palladium oder Cobalt ist.

24. Isomerisierungsverfahren nach Anspruch 23, wobei die ungesättigte organische Nitrilverbindung 2-Methyl-3-butennitril ist und das Metall der Gruppe VIII Nickel ist.

25. Isomerisierungsverfahren nach Anspruch 24, wobei das 2-Methyl-3-butennitril weniger als 100 ppm Peroxide enthält.

## Revendications

1. Procédé de préparation d'une composition polymère contenant du phosphore par:
(1) préparation d'un précurseur polymère par la polymérisation d'au moins un composé de formule I et/ou d'au moins un composé de formule II, 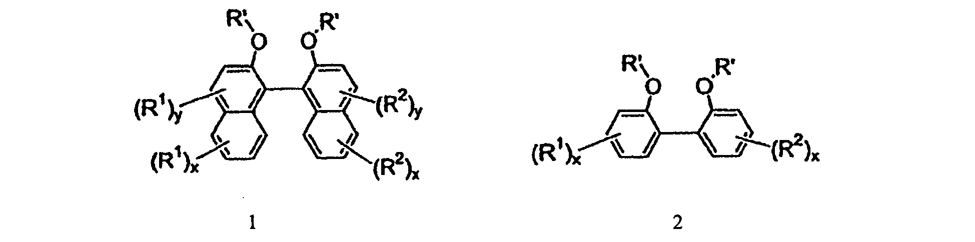 dans lesquelles:
x 0 à 4;
y 0 à 2;
et chaque R' est individuellement un atome d'hydrogène ou un groupe protecteur hydroxyle choisi parmi le groupe alkyle, alcoxyalkyle, carbonylalkyle, ou un éther couronne formé en prenant les deux groupes R' ensemble;
chaque R¹ et R² sont individuellement un atome d'hydrogène, un groupe alkyle linéaire ou ramifié, cycloalkyle, acétal, cétal, aryle, alcoxy, cycloalcoxy, aryloxy, ester, nitrile, fluor, chlore, brome, alkyle perhalogéné, hydrocarbylsulfinyle, hydrocarbylsulfonyle, formyle, hydrocarbylcarbonyle et éther cyclique;
à condition qu'au moins deux R¹ ou au moins deux R² ou au moins un R¹ et au moins un R² soient capables de réagir l'un avec l'autre pour entraîner le couplage aryle à aryle d'au moins un 2,2'-dihydroxyl-1,1'-binaphtalène substitué tel que montré dans la formule I et/ou un 2,2'-dihydroxyl-1,1'-biphénylène substitué tel que montré dans la formule II;
et dans lequel ledit couplage aryle à aryle est obtenu par:
i. polymérisation pour former une liaison carbone carbone liant au moins un composé de formule I et/ou au moins un composé de formule II; ou
ii. copolymérisation d'au moins un composé de formule I et/ou d'au moins un composé de formule II en la présence d'un comonomère aryle ayant au moins un R¹ et au moins un R² capables de réagir avec au moins un composé de formule I et/ou au moins un composé de formule II; ou
iii. copolymérisation d'au moins un composé de formule I et/ou d'au moins un composé de formule II par une réaction de Friedel-Crafts d'au moins un composé de formule I et/ou d'au moins un composé de formule II en la présence d'au moins un comonomère de dialkylation ou de dibenzylation ou de diacylation; ou
iv. copolymérisation d'au moins un composé de formule II avec un aldéhyde; et,
(2) si R' est un groupe protecteur hydroxyle, la conversion de R' en H, ou métal alcalin, ou alcalino-terreux et,
(3) phosphonylation du produit de l'étape (1) si R' est autre qu'un groupe protecteur hydroxyle, ou du produit des étapes (1) et (2) si R' est un groupe protecteur hydroxyle, avec au moins une diaryloxyphosphite [-P(-O-Ar)₂], une diarylphosphine [-P(Ar)₂], et/ou une aryl, aryloxyphosphinite [-P(Ar)(-O-Ar)];
où chaque Ar est individuellement un groupe phényle ou naphtyle, à condition que les deux groupes Ar qui sont directement ou indirectement liés au même atome de phosphore puissent être liés l'un à l'autre par un motif de liaison choisi parmi une liaison directe, alkylidène, amine secondaire ou tertiaire, oxygène, sulfure, sulfone, et sulfoxyde;
et chaque Ar peut en outre être substitué par un groupe alkyle en C₁ à C₂₀ à chaîne ramifiée ou linéaire, cycloalkyle en C₁ à C₂₀, aryle en C₆ à C₂₀, acétal, cétal, cycloalcoxy, aryloxy, alkyle perhalogéné, fluor, chlore, brome, formyle, ester, hydrocarbylsulfinyle, hydrocarbylsulfonyle, hydrocarbylcarbonyle, éther cyclique, -OR³, -CO₂R³, -SO₃R³, -S(O)R³, -SO₂R³, -CHO, -C(O)R³, ou CN; où chaque R³ est indépendamment un groupe alkyle en C₁ à C₂₀ à chaîne ramifiée ou linéaire, et cycloalkyle en C₁ à C₂₀, ou aryle en C₆ à C₂₀.

2. Composition polymère contenant du phosphore qui peut être obtenue par le procédé décrit dans la revendication 1.

3. Composition catalytique comprenant au moins une composition polymère contenant du phosphore selon la revendication 2 et au moins un métal du Groupe VIII.

4. Composition catalytique selon la revendication 3, où ledit métal du Groupe VIII est nickel, palladium, cobalt.

5. Composition catalytique selon la revendication 3, où ledit métal du Groupe VIII est rhodium, iridium et platine.

6. Composition catalytique selon la revendication 4, où ledit métal du Groupe VIII est le nickel.

7. Composition catalytique selon la revendication 4 ou la revendication 6, qui comprend également un acide de Lewis.

8. Composition catalytique selon la revendication 7, où ledit acide de Lewis est sélectionné parmi le groupe consistant en ZnBr₂, ZnI₂, ZnCl₂, ZnSO₄, CuCl₂, CuCl, Cu(O₃SCF₃)₂, CoCl₂, CoI₂, FeI₂, FeCl₃, FeCl₂, FeCl₂(THF)₂, TiCl₄(THF)₂, TiCl₂, ClTi(OiPr)₂, MnCl₂, ScCl₃, AlCl₃, (C₈H₁₇)AlCl₂, (C₈H₁₇)₂AlCl, (iso-C₄H₉)₂AlCl, Ph₂AlCl, PhAlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, (C₆H₅)₃SnX, où X = CF₃SO₃, CH₃C₆H₅SO₃ ou (C₆H₅)₃BCN, B(C₆H₅)₃, et TaCl₅.

9. Composition catalytique selon la revendication 8, où ledit acide de Lewis est chlorure de zinc ou chlorure de fer.

10. Processus d'hydrocyanation qui comprend la mise en contact d'un composé organique insaturé avec de l'HCN en présence d'une composition catalytique comprenant au moins une composition selon la revendication 2 et au moins un métal du Groupe VIII ainsi qu'un acide de Lewis en option.

11. Processus d'hydrocyanation selon la revendication 10, où ledit acide de Lewis est sélectionné parmi le groupe consistant en ZnBr₂, ZnI₂, ZnCl₂, ZnSO₄, CuCl₂, CuCl, Cu(O₃SCF₃)₂, CoCl₂, CoI₂, FeI₂, FeCl₃, FeCl₂, FeCl₂(THF)₂, TiCl₄(THF)₂, TiCl₂, ClTi(OiPr)₂, MnCl₂, ScCl₃, AlCl₃, (C₈H₁₇)AlCl₂, (C₈H₁₇)₂AlCl, (iso-C₄H₉)₂AlCl, Ph₂AlCl, PhAlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, (C₆H₅)SnX, où X = CF₃SO₃, CH₃C₆H₅SO₃, ou (C₆H₅)₃BCN, B(C₆H₅)₃, et TaCl₅.

12. Processus d'hydrocyanation selon la revendication 11, où ledit acide de Lewis est chlorure de zinc ou chlorure de fer.

13. Processus d'hydrocyanation selon la revendication 12, où ledit métal du Groupe VIII est nickel, palladium ou cobalt.

14. Processus d'hydrocyanation selon la revendication 13, où ledit composé organique insaturé est le 3-pentènenitrile ou le 4-pentènenitrile et ledit métal du Groupe VIII est le nickel.

15. Processus d'hydrocyanation selon la revendication 14, où le HCN contient moins de 20 ppm de dioxyde de soufre, moins de 40 ppm d'acide sulfurique, moins de 20 ppm de cyanogène, moins de 10 ppm d'époxyde, moins de 20 ppm d'acrylonitrile et moins de 100 ppm de peroxydes et lesdits pentènenitriles contiennent moins de 100 ppm de peroxydes.

16. Processus d'hydrocyanation selon la revendication 15, où ledit métal du Groupe VIII est le nickel et ledit composé organique insaturé est le 1,3-butadiène.

17. Processus d'hydrocyanation selon la revendication 16, où le HCN contient moins de 20 ppm de dioxyde de soufre, moins de 40 ppm d'acide sulfurique, moins de 20 ppm de cyanogène, moins de 10 ppm d'époxyde, moins de 20 ppm d'acrylonitrile et moins de 100 ppm de peroxydes et ledit 1,3-butadiène contient moins de 5 ppm de catéchol de t-butyle, moins de 500 ppm de cyclohexène de vinyle et moins de 100 ppm de peroxydes.

18. Processus d'hydroformylation qui comprend la mise en contact d'un composé organique insaturé avec du CO et de l'H₂ en présence d'une composition catalytique comprenant au moins une composition selon la revendication 2 et au moins un métal du Groupe VIII.

19. Processus d'hydroformylation selon la revendication 18, où ledit métal du Groupe VIII est rhodium, iridium ou platine.

20. Processus d'hydroformylation selon la revendication 19, où ledit composé organique insaturé est sélectionné parmi le groupe consistant en 3-pentènenitrile, acide 3-pentènoïque, 3-pentènal, alcool allylique, 3-pentènoate d'alkyle ou un mélange de ceux-ci.

21. Processus d'hydroformylation selon la revendication 20, où ledit composé organique insaturé contient moins de 100 ppm de peroxydes et ledit métal du Groupe VIII est le rhodium.

22. Processus d'isomérisation qui comprend la réaction d'un composé organique insaturé à fonction nitrile en présence d'une composition catalytique comprenant au moins une composition selon la revendication 2 et au moins un métal du Groupe VIII.

23. Processus d'isomérisation selon la revendication 22, où ledit métal du Groupe VIII est nickel, palladium ou cobalt.

24. Processus d'isomérisation selon la revendication 23, où ledit composé organique insaturé à fonction nitrile est le 2-méthyl-3-butènenitrile et ledit métal du Groupe VIII est le nickel.

25. Processus d'isomérisation selon la revendication 24, où le 2-méthyl-3-butènenitrile contient moins de 100 ppm de peroxydes.
